# EUROPÄISCHE PATENTSCHRIFT

(11) **EP 2 144 891 B1**
(45) Veröffentlichungstag und Bekanntmachung des Hinweises auf die Patenterteilung: **15.06.2016**
(21) Anmeldenummer: 08716677.3
(22) Anmeldetag: 22.03.2008
(51) Int. Cl.: C07D 261/12, A61K 31/42, A61K 31/422, A61K 31/423, C07D 261/20, C07D 413/04, C07D 413/12, C07D 413/14

(54) **5-OXO-ISOXAZOLE ALS INHIBITOREN VON LIPASEN UND PHOSPHOLIPASEN**
5-OXO-ISOXAZOLES AS INHIBITORS OF LIPASES AND PHOSPHOLIPASES
5-OXO-ISOXAZOLE EN TANT QU'INHIBITEURS DE LIPASES ET PHOSPHOLIPASES

(30) Priorität: 05.04.2007 EP 07007251
(43) Veröffentlichungstag der Anmeldung: 20.01.2010
(73) Patentinhaber: SANOFI, 75008 Paris (FR)
(72) Erfinder: PETRY, Stefan, 65926 Frankfurt am Main (DE); SEIDEL, Manfred, 65926 Frankfurt am Main (DE); ZOLLER, Gerhard, 65926 Frankfurt am Main (DE); MÜLLER, Günter, 65926 Frankfurt am Main (DE); BARINGHAUS, Karl-Heinz, 65926 Frankfurt am Main (DE); HEUER, Hubert, 65926 Frankfurt am Main (DE)
(74) Vertreter: Essler, Frank
(86) Internationale Anmeldenummer: PCT/EP2008/002314
(87) Internationale Veröffentlichungsnummer: WO 2008/122357

(56) Entgegenhaltungen:
- WO-A-2004/094393
- WO-A-2005/073199
- LOWE D B ET AL: "In vitro SAR of (5-(2H)-isoxazolonyl) ureas, potent inhibitors of hormone-sensitive lipase" BIOORGANIC & MEDICINAL CHEMISTRY LETTERS, OXFORD, GB, Bd. 14, Nr. 12, 21. Juni 2004 (2004-06-21), Seiten 3155-3159, XP004841364 ISSN: 0960-894X in der Anmeldung erwähnt

## Beschreibung

Die vorliegende Erfindung betrifft 5-Oxo-isoxazole der allgemeinen Formel I, deren pharmazeutisch anwendbare Salze und deren Verwendung als Arzneistoffe.

Bestimmte 3-Oxo-3H-benzo[c]isoxazole-1-carbonsäureamide sind beschrieben als Acylpeptidasehydrolase Inhibitoren in der WO 01/44211.

In Lowe et al., Bioorg. Med. Chem. Lett. 14 (2004) 3155-3159 werden 3-Oxoisoxazol-5-harnstoffderivate beschrieben, die wirksam an der hormonsensitiven Lipase sind. Ausgeführte Beispiele, welche Wasserstoff an der Harnstoffaminogruppe tragen werden als nicht wirksam an der hormonsensitiven Lipase beschrieben. Die genannten Beispiele sind auch an der endothelialen Lipase nicht wirksam.

Verbindungen mit hemmender Wirkung auf die endotheliale Lipase sind im Stand der Technik beispielsweise in der WO2004/094394, WO2004/094393, WO2004/093872 oder der WO2006/111321 beschrieben.

Der Erfindung lag die Aufgabe zugrunde, neue Verbindungen zur Verfügung zu stellen, die eine therapeutisch verwertbare Wirkung entfalten. Insbesondere bestand die Aufgabe darin, neue Verbindungen zu finden, die zur Behandlung von erhöhten Lipidkonzentrationen im Blut, dem metabolischen Syndrom, Diabetes, Insulinresistenz, der Dysregulation von LDL, HDL oder Herzkreislauferkrankungen geeignet sind.

Aufgabe der vorliegenden Erfindung ist die Bereitstellung von Verbindungen, die eine Hemmung der endothelialen Lipase bewirken.

Gegenstand der Erfindung sind 5-Oxo-isoxazole der allgemeinen Formel I 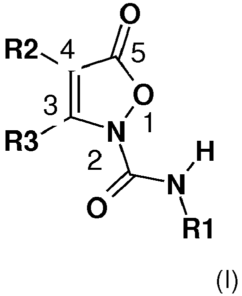 wobei bedeuten:
- R1: Y-Aryl, Y-Heteroaryl, wobei Aryl oder Heteroaryl ein oder mehrfach durch, F, Cl, Br, l, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₃-C₈)-Cycloalkyl, O-(C₃-C₈)-Cycloalkyl, (C₂-C₆)-Alkinyl, (C₀-C₈)-Alkylen-Aryl, O-(C₀-C₈)-Alkylen-Aryl, S-Aryl, (C₀-C₈)-Alkylen-Heteroaryl, N(R4)(R5), SO₂-CH₃, SO₂-NH₂, SF₅, COOH, COO-(C₁-C₆)-Alkyl, CON(R6)(R7), N(R8)CO(R9), N(R10)SO₂(R11), CO(R12), (CR13R14)ₓ-O(R15), O-CO-N(R16)(R17), O-CO-(C₁-C₆)-Alkylen-CO-O-(C₁-C₆)-Alkyl, O-CO-(C₁-C₆)-Alkylen-CO-OH, O-CO-( C₁-C₆)-Alkylen-CO-N(R18)(R19) substituiert sein kann, wobei Aryl oder Heteroaryl wiederum durch
F, Cl, Br, l, OH, CF₃, NO₂, CN, OCF₃, O-(C₁-C₆)-Alkyl, O-(C₁- C₄)-Alkoxy-(C₁-C₄)-alkyl, S-(C₁-C₆)-Alkyl, (C₁-C₆)-Alkyl, (C₂-C₄)- Haloalkyl, (C₂-C₆)-Alkenyl, (C₃-C₈)-Cycloalkyl, O-(C₃-C₈)- Cycloalkyl, (C₂-C₆)-Alkinyl, N(R4a)(R5a), SO₂-CH₃, SF₅, COOH, COO-(C₁-C₆)-Alkyl, CON(R6a)(R7a), N(R8a)CO(R9a), N(R10a)SO₂(R11a), CO(R12a), (CR13aR14a)ₓ-O(R15a), O- CO-N(R16a)(R17a), O-CO-(C₁-C₆)-Alkylen-CO-O-(C₁-C₆)- Alkyl, O-CO-( C₁-C₆)-Alkylen-CO-OH, O-CO-(C₁-C₆)-Alkylen- CO-N(R18a)(R19a) ein oder mehrfach substituiert sein kann;
- x, x': 0, 1, 2, 3, 4, 5, 6;
R4, R5, R6, R7, R9, R10, R11, R12, R13, R14, R15, R16, R17, R18, R19, R4a, R5a, R6a, R7a, R9a, R10a, R11a, R12a, R13a, R14a, R15a, R16a, R17a, R18a, R19a
unabhängig voneinander Wasserstoff, (C₁-C₈)-Alkyl;
- Y, Z: gleich oder verschieden (C₁-C₂)-Alkylen, welches durch F, Cl, CH₃ oder OH einfach substituiert sein kann;
- R2: Wasserstoff, (C₁-C₁₂)-Alkyl, Z-Aryl, wobei Aryl gegebenenfalls substituiert sein kann, (C₃-C₁₂)-Cycloalkyl;
- R3: (C₁-C₁₂)-Alkyl, Aryl, Heteroaryl, , wobei Aryl oder Heteroaryl gegebenenfalls substituiert sein kann, (C₃-C₁₂)-Cycloalkyl; oder
- R2 und R3: zusammen mit dem sie tragenden Kohlenstoffatomen bilden ein monocyclisches, gesättigtes oder teilweise ungesättigtes 4- bis 8-gliedriges Ringsystem, deren einzelne Glieder durch ein bis drei Atome oder Atomgruppen aus der Reihe -CHR64-, -CR64R65-, =(C-R66)-,-NR67-, -C(=O)-, -O- ersetzt sein können, mit der Maßgabe, dass zwei Einheiten aus der Reihe -O- nicht benachbart sein dürfen;
- R64, R65, R66, R67: gleich oder verschieden Wasserstoff, F, Cl, Br, l, OH, CF₃, NO₂, CN, OCF₃, SF₅, O-(C₁-C₆)-Alkyl, O-(C₁-C₄)-Alkoxy-(C₁- C₄)-Alkyl, S-(C₁-C₆)-Alkyl, (C₁-C₆)-Alkyl, (C₂-C₄)-Haloalkyl, O- (C₂-C₄)-Haloalkyl, (C₂-C₆)-Alkenyl, (C₃-C₈)-Cycloalkyl, O-(C₃- C₈)-Cycloalkyl, (C₃-C₈)-Cycloalkenyl, (C₂-C₆)-Alkinyl, N(R68)(R69), SO₂-CH₃, COOH, COO-(C₁-C₆)-Alkyl, CON(R70)(R71), N(R72)CO(R73), N(R74)SO₂(R75), CO(R76), (CR77R78)_{x''''}- O(R79), O-CO-N(R80)(R81), O-CO-(C₁-C₆)- Alkylen-CO-O-(C₁-C₆)-Alkyl, O-CO-( C₁-C₆)-Alkylen-CO-OH, O- CO-(C₁-C₆)-Alkylen-CO-N(R82)(R83);
- x'''': 0, 1, 2, 3, 4, 5, 6;
R68, R69, R70, R71, R72, R73, R74, R75, R76, R77, R78, R79, R80, R81, R82, R83
gleich oder verschieden Wasserstoff, (C₁-C₆)-Alkyl;
die tautomeren Formen der Verbindung sowie deren physiologisch verträgliche Salze.

Bevorzugt sind Verbindungen der Formel I, worin
- R1: Y-Phenyl, Y-Heteroaryl, wobei Heteroaryl 1 Heteroatom aus der Reihe N, O, S enthält und wobei Phenyl oder Heteroaryl durch F, Cl, Br, OH, CF₃, NO₂, CN, OCF₃, O-(C₁-C₆)-Alkyl, S-(C₁-C₆)-Alkyl, (C₁-C₆)-Alkyl, (C₂-C₄)-Haloalkyl, (C₃-C₈)-Cycloalkyl, (C₀-C₆)-Alkylen-Phenyl, O-(C₀-C₆)-Alkylen-Phenyl, S-Phenyl, (C₀-C₈-Alkylen-Heteroaryl, N(R4)(R5), COOH, COO-(C₁-C₆)-Alkyl, CON(R6)(R7), CO(R12), ein- oder mehrfach substituiert sein kann, wobei Phenyl oder Heteroaryl wiederum durch
F, Cl, OH, CF₃, NO₂, CN, OCF₃, O-(C₁-C₆)-Alkyl, S-(C₁-C₆)- Alkyl, (C₁-C₆)-Alkyl, (C₂-C₄)-Haloalkyl, (C₃-C₈)-Cycloalkyl, N(R4a)(R5a), COOH, COO-(C₁-C₆)-Alkyl, CON(R6a)(R7a) CO(R12a) ein oder mehrfach substituiert sein kann;
- x, x': 0, 1, 2, 3, 4, 5, 6;
R4, R5, R6, R7, R12, R4a, R5a, R6a, R7a, , R12a,
gleich oder verschieden Wasserstoff, (C₁-C₈)-Alkyl;

- Y, Z: gleich oder verschieden -CH₂- oder -CH₂-CH₂-, welches durch CH₃ oder OH einfach substituiert sein kann;
- R2: Wasserstoff, (C₁-C₁₂)-Alkyl, Z-Phenyl, wobei Phenyl gegebenenfalls substituiert sein kann, (C₃-C₁₂)-Cycloalkyl;
- R3: (C₁-C₁₂)-Alkyl, Phenyl, Heteroaryl, welches 1 Heteroatom aus der Reihe N, O, S enthält, wobei Phenyl oder Heteroaryl gegebenenfalls substituiert sein kann, (C₃-C₁₂)-Cycloalkyl; oder
- R2 und R3: zusammen mit dem sie tragenden Kohlenstoffatomen bilden ein monocyclisches, gesättigtes 5- bis 7-gliedriges Ringsystem, dessen einzelne Glieder durch ein bis drei Atomgruppen aus der Reihe -CHR64-, -CR64R65-, =(C-R66)- ersetzt sein können;
- R64, R65,: R66 gleich oder verschieden F, Cl, OH, CF₃, O-(C₁-C₆)-Alkyl, O-(C₁- C₄)-Alkoxy-(C₁-C₄)-Alkyl, (C₁-C₆)-Alkyl, (C₂-C₄)-Haloalkyl, (C₃- C₈)-Cycloalkyl, N(R68)(R69), SO₂-CH₃, COOH, COO-(C₁-C₆)- Alkyl, CON(R70)(R71), N(R72)CO(R73), CO(R76), O-CO- N(R80)(R81), O-CO-(C₁-C₆)-Alkylen-CO-O-(C₁-C₆)-Alkyl, O- CO-(C₁-C₆)-Alkylen-CO-OH, O-CO-(C₁-C₆)-Alkylen-CO- N(R82)(R83);
R68, R69, R70, R71, R72, R73, R76, R77, R78, R79, R80, R81, R82, R83
unabhängig voneinander Wasserstoff, (C₁-C₆)-Alkyl;
bedeuten,
die tautomeren Formen der Verbindungen sowie deren physiologisch verträgliche Salze.

Besonders bevorzugt sind Verbindungen der Formel I, worin
- R1: Y-Phenyl, Y-Pyridyl, Y-Thienyl, Y-Furyl, Y-Benzothienyl, Y-Benzofuryl, wobei Phenyl oder der heteroaromatische Rest durch F, Cl, Br, CF₃, CN, OCF₃, O-(C₁-C₆)-Alkyl, (C₁-C₆)-Alkyl, (C₃-C₆)-Cycloalkyl, N(R4)(R5), COOH, COO-(C₁-C₆)-Alkyl, CON(R6)(R7), CO(R12) ein-, zwei- oder dreifach substituiert und durch (C₀-C₁)-Alkylen-Phenyl, O-(C₀-C₁)-Phenyl, Pyrazolyl, Pyridyl, Thienyl, Furyl, Benzothienyl, Benzofuryl einfach substituiert sein kann, wobei ein heteroaromatische Rest oder Phenyl wiederum durch F, Cl, Br, CF₃, CN, OCF₃, O-(C₁-C₆)-Alkyl, (C₁-C₆)-Alkyl, (C₃-C₆)-Cycloalkyl, N(R4a)(R5a), COOH, COO-(C₁-C₆)-Alkyl, CON(R6a)(R7a), CO(R12a) ein-, zwei oder dreifach substituiert sein kann;

- R4, R5, R6, R7, R12, R4a, R5a, R6a, R7a, R12a: unabhängig voneinander H, (C₁-C₈)-Alkyl;
- Y: -CH₂- oder -CH₂-CH₂-, welches durch CH₃ einfach substituiert sein kann;
- R2: Wasserstoff, (C₁-C₈)-Alkyl, -CH₂-Phenyl, wobei Phenyl gegebenenfalls substituiert sein kann, (C₃-C₈)-Cycloalkyl;
- R3: (C₁-C₆)-Alkyl, Phenyl, Pyridyl, Thienyl, wobei Phenyl, Pyridyl oder Thienyl gegebenenfalls substituiert sein kann, (C₃-C₈)-Cycloalkyl; oder
- R2 und R3: zusammen mit dem sie tragenden Kohlenstoffatomen bilden ein monocyclisches, gesättigtes 6- bis 7-gliedriges Ringsystem, deren einzelne Glieder durch ein bis drei Atome oder Atomgruppen aus der Reihe -CHR64-, -CR64R65-, ersetzt sein können;
- R64, R65: gleich oder verschieden F, Cl, CF₃, OCF₃, (C₁-C₆)-Alkyl, O-(C₁-C₆)-Alkyl, N(R68)(R69), COOH, COO-(C₁-C₆)-Alkyl, CO-N(R70)(R71), CO(R76);
- R68, R69, R70, R71, R76: gleich oder verschieden Wasserstoff, (C₁-C₆)-Alkyl;
bedeuten,
die tautomeren Formen der Verbindungen sowie deren physiologisch verträgliche Salze.

Bei einer besonders bevorzugten Ausführungsform der Verbindungen der Formel I ist
- R2: Isopropyl und
- R3: Methyl.

Bei einer weiteren besonders bevorzugten Ausführungsform der Verbindungen der Formel I ist
- R2: Wasserstoff und
- R3: Phenyl, welches durch Cl einfach substituiert sein kann.

Bei einer weiteren besonders bevorzugten Ausführungsform der Verbindungen der Formel I bedeuten
R2 und R3 zusammen -CH₂-CH₂-CH₂-CH₂- oder -CH₂-CH₂-CH₂-CH₂-CH₂-.

Ganz besonders bevorzugt sind Verbindungen der Formel I, worin
- R1: Y-Phenyl, Y-Thienyl, Y-Benzothienyl, wobei Phenyl oder der heteroaromatische Rest durch F, Cl, Br, CF₃, O-CH₃, -CH₃, -CH₂CH₃,-CH₂-CH₂-CH₂-CH₃, ein-, zwei- oder dreifach substituiert und durch Phenyl, Pyrazolyl oder Thienyl einfach substituiert sein kann, wobei der heteroaromatische Rest oder Phenyl wiederum durch F, Cl, Br, CF₃, O-(C₁-C₆)-Alkyl, (C₁-C₆)-Alkyl ein-, zwei- oder dreifach substituiert sein kann;
- Y: -CH₂-, welches durch CH₃ einfach substituiert sein kann;
- R2: Wasserstoff, Methyl, Isopropyl, Cyclopropyl, -CH₂-Phenyl, wobei Phenyl durch Cl in 4-Position substituiert sein kann;
- R3: Methyl, Phenyl, Pyridyl, Cyclopropyl, wobei Phenyl durch Cl substituiert sein kann; oder
- R2 und R3: zusammen -CH₂--CH₂-CH₂-CH₂- oder -CH₂-CH₂-CH₂-CH₂-CH₂;
bedeuten,
die tautomeren Formen der Verbindungen sowie deren physiologisch verträgliche Salze.

Die Erfindung bezieht sich auf Verbindungen der Formel I, in Form ihrer Salze, Racemate, racemischen Mischungen und reinen Enantiomere sowie auf ihre Diastereomere und Mischungen davon.

Die Alkylreste in den Substituenten R1, R2, R3, R4, R5, R6, R7, R9, R10, R11, R12, R13, R14, R15, R16, R17, R18, R19, R4a, R5a, R6a, R7a, R9a, R10a, R11 a, R12a, R13a, R14a, R15a, R16a, R17a, R18a, R19a, R64, R65, R66, R67, R68, R69, R70, R71, R72, R73, R74, R75, R76, R77, R78, R79, R80, R81, R82, R83 können sowohl geradkettig wie verzweigt sein.

Halogen steht für Fluor, Chlor, Brom oder lod, besonders für Fluor oder Chlor. Unter Haloalkyl wird ein durch Halogen ein oder mehrfach substituierter Alkylrest verstanden.

Unter einem Arylrest wird ein Phenyl-, Naphthyl- oder Biphenylrest verstanden. Bevorzugter Arylrest ist Phenyl.
Die Arylreste können ein oder mehrfach mit geeigneten Gruppen substituiert sein, wie z.B.: F, Cl, Br, l, CF₃, OH, OCF₃, NO₂, CN, COOH, COO(C₁-C₆)Alkyl, CONH₂, CONH(C₁-C₆)Alkyl, CON[(C₁-C₆)Alkyl]₂, , (C₃-C₁₀)-Cycloalkyl, (C₁-C₁₀)-Alkyl, (C₂-C₆)-Alkenyl, (C₂-C₆)-Alkinyl, O-(C₁-C₆)-Alkyl, CO-(C₁-C₆)-Alkyl, O-CO-(C₁-C₆)-Alkyl, O-CO-(C₁-C₆)-Aryl,
PO₃H₂, SO₃H, SO₂-NH₂, SO₂NH(C₁-C₆)-Alkyl SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH2)ₙ-Heterocyclus, SO₂-N((CH₂)ₙ-Aryl)₂, SO₂-N((CH₂)ₙ-(Heterocyclus)₂, NH-(CH₂)ₙ-Aryl, NH-(CH₂)ₙ-Heterocyclus, N((C₁-C₆)-Alkyl)(CH₂)ₙ-Aryl, N((C₁-C₆)-Alkyl)(CH₂)ₙ-Heterocyclus,
C(NH)(NH₂), NH₂, NH-(C₁-C₆)-Alkyl, N((C₁-C₆)-Alkyl)₂, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, l, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂, wobei Aryl ohne den Zusatz "gegebenenfalls substituiert sein kann" unsubstituiertes Aryl bedeutet.

Heterocyclus ist ein mono- oder bicyclisches Ringsystem mit 5 bis 12 Ringgliedern, worin mindestens ein Atom im Ringsystem ein Heteroatom aus der Reihe N, O und S ist. Ferner gehören auch Ringsysteme zu dieser Definition, worin der Heterocyclus mit einem Benzolkern kondensiert ist. (C₅-C₇)-Heterocyclus ist ein monozyklisches, (C₈-C₁₂)-Heterocyclus ein bicyclisches Ringsystem.
Geeignete "Heterocyclische Ringe" bzw. "Heterocyclische Reste" sind Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, Furanyl, Furazanyl, Imidazolidinyl, Imidazolinyl, Imidazolyl, 1 H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazolyl, Tetrazolyl und Xanthenyl.
Pyridyl steht sowohl für 2-, 3- als auch 4-Pyridyl. Thienyl steht sowohl für 2- als auch 3-Thienyl. Furyl steht sowohl für 2- als auch 3-Furyl.

Umfasst sind weiterhin die entsprechenden N-Oxide dieser Verbindungen, also z.B. 1-Oxy-2-, 3- oder 4-pyridyl.

Heteroaryl ist eine Untergruppe von Heterocyclus und ist ein mono- oder bicyclisches aromatisches Ringsystem mit 5 bis 12 Ringgliedern, worin mindestens ein Atom im Ringsystem ein Heteroatom aus der Reihe N, O und S ist.
Geeignete "Heteroaryl-Ringe" bzw. "Heteroaryl-Reste" sind beispielsweise Benzimidazolyl, Benzofuranyl, Benzothienyl, Benzoxazolyl, Benzthiazolyl, Benztriazolyl, Benztetrazolyl, Benzisoxazolyl, Benzisothiazolyl, Chinolinyl, Furanyl, Furazanyl, Imidazolyl, 1 H-Indazolyl, Indolyl, 1,2,3-Oxadiazolyl, 1,2,4-Oxadiazolyl, 1,2,5-Oxadiazolyl, 1,3,4-Oxadiazolyl, Pyrimidinyl, Pyrazinyl, Pyrazolyl, Pyridyl, Pyrrolyl, Thiazolyl, 1,2,3-Thiadiazolyl, 1,2,4-Thiadiazolyl, 1,2,5-Thiadiazolyl, 1,3,4-Thiadiazolyl, Thienyl.
Bevorzugte Heteroarylreste sind Thienyl, Pyridyl, Furanyl, Pyrazolyl, Benzothienyl und Benzofuranyl. Besonders bevorzugte Heteroaryl-Reste sind Thienyl, Benzothienyl und Furanyl, insbesondere bevorzugt ist Thienyl.

Die Heterocyclischen Reste, bzw. die Heteroaromatischen Reste können ein oder mehrfach mit geeigneten Gruppen substituiert sein, wie z.B. F, Cl, Br, l, CF₃, OH, OCF₃, NO₂, CN, COOH, COO(C₁-C₆)Alkyl, CONH₂, CONH(C₁-C₆)Alkyl, CON[(C₁-C₆)Alkyl]₂, , (C₃-C₁₀)-Cycloalkyl, (C₁-C₁₀)-Alkyl, (C₂-C₆)-Alkenyl, (C₂-C₆)-Alkinyl, O-(C₁-C₆)-Alkyl, CO-(C₁-C₆)-Alkyl, O-CO-(C₁-C₆)-Alkyl, O-CO-(C₁-C₆)-Aryl, PO₃H₂, SO₃H, SO₂-NH₂, SO₂NH(C₁-C₆)-Alk SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, SO₂-N((CH₂)ₙ-(Heterocyclus)₂, NH-(CH₂)ₙ-Aryl, NH-(CH₂)ₙ-Heterocyclus, N((C₁-C₆)-Alkyl)(CH₂)ₙ-Aryl, N((C₁-C₆)-Alkyl)(CH₂)ₙ-Heterocyclus, C(NH)(NH₂), NH₂, NH-(C₁-C₆)-Alkyl, N((C₁-C₆)-Alkyl)₂, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂, wobei Heterocyclus oder Heteroaryl ohne den Zusatz "gegebenenfalls substituiert sein kann" unsubstituierten Heterocyclus oder Heteroaryl bedeutet.

Unter einem Cycloalkylrest wird ein einen oder mehrere Ringe enthaltendes Ringssystem, welches gesättigt vorliegt, verstanden, das ausschließlich aus Kohlenstoffatomen aufgebaut ist, wie z.B. Cyclopropyl, Cyclopentyl, Cyclohexyl oder Adamantyl.
Die Cycloalkylrestereste können ein oder mehrfach mit geeigneten Gruppen substituiert sein, wie z.B.: F, Cl, Br, l, CF₃, OH, OCF₃, NO₂, CN, COOH, COO(C₁-C₆)Alkyl, CONH₂, CONH(C₁-C₆)Alkyl, CON[(C₁-C₆)Alkyl]₂, , (C₃-C₁₀)-Cycloalkyl, (C₁-C₁₀)-Alkyl, (C₂-C₆)-Alkenyl, (C₂-C₆)-Alkinyl, O-(C₁-C₆)-Alkyl, CO-(C₁-C₆)-Alkyl, O-CO-(C₁-C₆)-Alkyl, O-CO-(C₁-C₆)-Aryl,
PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, SO₂-N((CH₂)ₙ-(Heterocyclus)₂, NH-(CH₂)ₙ-Aryl, NH-(CH₂)ₙ-Heterocyclus, N((C₁-C₆)-Alkyl)(CH₂)ₙ-Aryl, N((C₁-C₆)-Alkyl)(CH₂)ₙ-Heterocyclus,
C(NH)(NH₂), NH₂, NH-(C₁-C₆)-Alkyl, N((C₁-C₆)-Alkyl)₂, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂, wobei Cycloalkyl ohne den Zusatz "gegebenenfalls substituiert sein kann" unsubstituiertes Cycloalkyl bedeutet.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze) und Salze von Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I "auf Verbindung(en) der Formel I, wie vorstehend beschrieben, sowie ihre Salze, wie hierin beschrieben.

### Verwendung

Die erfindungsgemäßen Verbindungen der allgemeinen Formel I besitzen eine überraschende hemmende Wirkung auf die endotheliale Lipase (EL). Das antiatheroskleotisch wirksame HDL ist das bevorzugte Substrat für EL. Eine Senkung des HDL-Spiegels führt zur Progression von Atherosklerose und ihrer Folgeerkrankungen wie Metabolisches Syndrom und Koronare Herzerkrankungen. Eine Hemmung der EL sollte somit zur Vorbeugung von atherosklerotischen Erkrankungen führen.
Weiterhin wurde gefunden, dass die hemmende Wirkung der erfindungsgemäßen Verbindungen der allgemeinen Formel I selektiv gegenüber anderen Lipasen, wie beispielsweise der hormonsensitiven Lipase (HSL) ist.

Die Verbindungen der Formel I zeigen eine außerdem eine verbesserte Löslichkeit in wässrigen Medien bei mindestens gleich hoher Aktivität, verglichen mit Verbindungen ähnlicher Strukturen. Die erfindungsgemäßen Verbindungen zeichnen sich weiterhin durch weitere vorteilhafte Eigenschaften, wie höhere metabolische Stabilität und Serumstabilität gegenüber Verbindungen des Standes der Technik aus.

Besonders geeignet sind solche Verbindungen zur Behandlung und/oder Prävention von
1. Dyslipidämien und allgemeine Störungen des Fettstoffwechsels und deren Folgen, wie z.B. Atherosklerose, koronare Herzkrankheit, zerebrovaskuläre Erkrankungen etc, insbesondere solche (aber nicht beschränkt auf), die durch einen oder mehrerer folgender Faktoren charakterisiert sind:
   - hohe Plasma-Triglycerid-, hohe postprandiale Plasma-Triglycerid-Konzentrationen
   - niedrige HDL-Cholesterin Konzentration
   - niedrige ApoA Lipoprotein-Konzentrationen
   - hohe LDL-Cholesterin Konzentrationen
   - kleine dichte LDL-Cholesterin Partikel
   - hohe ApoB Lipoprotein-Konzentrationen
2. Verschiedenen andere Zuständen, die mit dem Metabolischen Syndrom assoziert sein können, sind wie:
   - Adipositas (Fettsucht), einschließlich abdominale Adipositas
   - Thrombosen, Stadien von Hyperkoagulabilität und Thromboseneigung (arteriell und venös)
   - Hoher Blutdruck
   - Herzinsuffizienz, wie z.B. (aber nicht beschränkt auf) bei Zustand nach Myokardinfarkt, hypertensive Herzerkrankung oder Kardiomyopathie
   - Diabetes mellitus, insbesondere Typ 2 Diabetes einschließlich der Verhinderung der damit verbundenen Folgeerkrankungen (Hyperglykämie, Glucoseintoleranz, Verlust der ß-Zellen der Bauchspeicheldrüse, makro- und mikrovaskulärer Erkrankungen
3. weitere Krankheiten oder Zustände bei welchen zum Beispiel entzündliche Reaktionen oder die Zelldifferenzierung eine Rolle spielt sind:
   - Atherosklerose wie z.B. (aber nicht beschränkt auf) Koronarsklerose einschl. Angina pectoris oder Herzinfarkt, Hirnschlag
   - Vaskuläre Restenose oder Reverschluß
   - Chronisch entzündliche Darmerkrankungen, wie z.B. Morbus Crohn und Colitis ulcerosa
   - Pankreatitis
   - Andere entzündliche Zustände
   - Retinopathie
   - Fettzell-Tumore (adipose cell tumors)
   - Fettzell-Karzinome, wie z.B. Liposarkome
   - solide Tumoren und Neoplasien, wie z.B. (aber nicht beschränkt auf) Karzinome des Magen-Darm Traktes, der Leber, der Gallenwege und des Pankreas, endokrine Tumore, Karzinome der Lunge, der Niere und harnableitenden Organe, des Genitaltraktes, Prostata-Karzinome etc.
   - akute und chronische myeloprolifeative Erkrankungen und Lymphome
   - Angiogenese
   - Neurodegenerative Erkrankungen
   - Alzheimersche Krankheit
   - Multiple Sklerose
   - Morbus Parkinson
   - Erythemato-squamöse Dermatosen, wie z.B. Psoriasis (Schuppenflechte)
   - Akne vulgaris
   - Andere Hautkrankheiten und dermatologische Zustände, die durch PPAR moduliert werden
   - Ekzeme und Neurodermitis
   - Dermatitiden, wie z.B. seborrhoische Dermatitis oder Lichtdermatitis
   - Keratitis und Keratosen, wie z.B. seborrhoische Keratosen, senile Keratosen, aktinische Keratose, photo-induzierte Keratosen oder Keratosis follicularis
   - Keloide und Keloid-Prophylaxe
   - Warzen, einschließlich Kondylomata oder Kondylomata acuminata
   - Human papilloma viral (HPV) Infektionen, wie z.B. venerische Papillomata, virale Warzen, wie z.B. Molluscum contagiosum, Leukoplakie
   - Papulöse Dermatosen, wie z.B. Lichen planus
   - Hautkrebs, wie z.B. Basalzellkarzinome, Melanome oder kutane T-Zell Lymphome
   - Lokalisierte, benigne epidermale Tumore, wie z.B. Keratoderma, epidermale Naevi
   - Frostbeulen
   - Hoher Blutdruck
   - Syndrom X
   - Syndrom der polyzystischen Ovarien (PCOS)
   - Asthma
   - Osteoarthritis
   - Lupus erythematodes (LE) oder entzündliche rheumatische Erkrankungen, wie z.B. Rheumatoide Arthritis
   - Vaskulitis
   - Auszehrung (Kachexie)
   - Gicht
   - Ischämie/Reperfusions Syndrom
   - Akutes respiratorisches Distress Syndrom (ARDS) ("Schocklunge")

### Galenik

Die Menge einer erfindungsgemäßen Verbindung, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 0.05 bis 1000 mg, typischerweise von 0,5 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer erfindungsgemäßer Verbindungen. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mitteln in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, welche die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die Verbindungen der Formel I zeichnen sich durch günstige Wirkungen auf Stoffwechselstörungen aus. Sie beeinflussen den Fett- und Zuckerstoffwechsel positiv, sie senken insbesondere den Triglyceridspiegel und sind zur Prävention und Behandlung von Typ II Diabetes und Arteriosklerose sowie deren vielfältigen Folgeerkrankungen geeignet.

### Kombinationen mit anderen Medikamenten

Die erfindungsgemäßen Verbindungen können alleine oder in Kombination mit einem oder mehreren weiteren pharmakologischen Wirkstoffen verabreicht werden. Insbesondere können die erfindungsgemäßen Verbindungen mit Wirkstoffen, die eine ähnliche pharmakologische Wirkung wie sie selbst aufweisen, verabreicht werden. Beispielsweise können sie in Kombination mit Wirkstoffen, die eine günstige Wirkungen auf Stoffwechselstörungen bzw. häufig damit assoziierte Erkrankungen haben, verabreicht werden. Beispiele für solche Medikamente sind
1. blutzuckersenkende Medikamente, Antidiabetika,
2. Wirkstoffe zur Behandlung von Dyslipidämien,
3. antiatherosklerotische Medikamente,
4. Mittel gegen Obesitas,
5. entzündungshemmende Wirkstoffe
6. Wirkstoffe zur Behandlung von malignen Tumoren
7. antithrombotische Wirkstoffe
8. Wirkstoffe zur Behandlung von Bluthochdruck
9. Wirkstoffe zur Behandlung von Herzinsuffizienz und
10. Wirkstoffe zur Behandlung und/oder Prävention von durch Diabetes verursachten bzw. mit Diabetes assoziierten Komplikationen.
11. Wirkstoffe zur Behandlung von neurodegenerativen Krankheiten
12. Wirkstoffe zur Behandlung von Krankheiten des Zentralen Nervensystems
13. Wirkstoffe zur Behandlung von Drogen-, Nikotin- und Alkoholabhängigkeit
14. Schmerzmittel

Sie lassen sich mit den erfindungsgemäßen Verbindungen der Formel I insbesondere zur synergistischen Wirkungsverbesserung kombinieren. Die Verabreichung der Wirkstoffkombination kann entweder durch getrennte Verabreichung der Wirkstoffe an den Patienten oder in Form von Kombinationsprodukten, bei denen mehrere Wirkstoffe in einer pharmazeutischen Zubereitung vorhanden sind, erfolgen.

Als weitere Wirkstoffe für die Kombinationspräparate sind insbesondere geeignet: Alle Antidiabetika, die in der Roten Liste 2006, Kapitel 12 genannt sind; alle Abmagerungsmittel/Appetitzügler, die in der Roten Liste 2006, Kapitel 1 genannt sind; alle Lipidsenker, die in der Roten Liste 2006, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus^{®} (siehe www.lantus.com) oder Apidra^{®} oder solche, wie sie in WO2005005477 (Novo Nordisk) beschrieben sind, schnell wirkende Insuline (siehe US 6,221,633), inhalierbare Insuline, wie z. B. Exubera ^{®} oder orale Insuline, wie z. B. IN-105 (Nobex) oder Orallyn™ (Generex Biotechnology), GLP-1-Derivate wie z.B. Exenatide, Liraglutide oder diejenigen die in WO 98/08871 oder WO2005/027978 von Novo Nordisk A/S, in WO 01/04156 von Zealand oder in WO 00/34331 von Beaufour-Ipsen offenbart wurden, Pramlintide Acetat (Symlin; Amylin Pharmaceuticals), sowie oral wirksame hypoglykämische Wirkstoffe.

Die Wirkstoffe umfassen vorzugsweise
Sulfonylharnstoffe,
Biguanide,
Meglitinide,
Oxadiazolidindione,
Thiazolidindione,
Glukosidase-Inhibitoren,
Hemmstoffe der Glykogen Phosphorylase,
Glukagon-Antagonisten,
Glukokinase Aktivatoren,
Inhibitoren der Fructose-1,6-bisphosphatase,
Modulatoren des Glukosetransporters-4 (GLUT4),
Inhibitoren der Glutamin-Fructose-6-Phosphat-Amidotransferase (GFAT), GLP-1-Agonisten,
Kaliumkanalöffner, wie z.B. diejenigen, die in WO 97/26265 und WO 99/03861 von Novo Nordisk A/S offenbart wurden,
Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption,
Hemmstoffe der 11ß-HSD1,
Inhibitoren der Protein-Tyrosin-Phosphatase-1 B (PTP1 B),
Modulatoren des natrium-abhängigen Glukosetransporters 1 oder 2 (SGLT1, SGLT2), den Fettstoffwechsel verändernde Verbindungen wie antihyperlipidämische Wirkstoffe und antilipidämische Wirkstoffe,
Verbindungen, die die Nahrungsmitteleinnahme verringern,
Verbindungen, die die Thermogenese erhöhen,
PPAR- und RXR-Modulatoren und
Wirkstoffe, die auf den ATP-abhängigen Kaliumkanal der Betazellen wirken.

Bei einer Ausführungsform der Erfindung wird die Verbindung der Formel I in Kombination mit einem HMGCoA-Reduktase Inhibitor, wie Simvastatin, Fluvastatin, Pravastatin, Lovastatin, Atorvastatin, Cerivastatin, Rosuvastatin oder L-659699, verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692), MD-0727 (Microbia Inc., WO2005021497) oder mit Verbindungen, wie in WO2002066464 (Kotobuki Pharmaceutical Co. Ltd.), WO2005062824 (Merck & Co.) oder WO2005061451 und WO2005061452 (AstraZeneca AB) beschrieben, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, Gl 262570, R-483 oder CS-011 (Rivoglitazon), verabreicht.

Bei einer Ausführungsform der Erfindung wird die Verbindung der Formel I in Kombination mit einem PPAR alpha Agonisten, wie z.B. GW9578, GW-590735, K-111, LY-674, KRP-101 oder DRF-10945, verabreicht.

Bei einer Ausführungsform der Erfindung wird die Verbindung der Formel I in Kombination mit einem gemischten PPAR alpha/gamma Agonisten, wie z.B. Muraglitazar, Tesaglitazar, Naveglitazar, LY-510929, ONO-5129, E-3030 oder wie in WO00/64888, WO00/64876, WO03/020269, WO2004075891, WO2004076402, WO2004075815, WO2004076447, WO2004076428, WO2004076401, WO2004076426, WO2004076427, WO2006018118, WO2006018115 und WO2006018116 oder in J.P. Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516, oder wie in WO2005097762, WO2005097786, WO2005097763, WO2006029699 beschrieben verabreicht.

Bei einer Ausführungsform der Erfindung wird die Verbindung der Formel I in Kombination mit Metaglidasen oder mit MBX-2044 oder anderen partiellen PPAR gamma Agonisten/Antagonisten verabreicht.

Bei einer Ausführungsform der Erfindung wird die Verbindung der Formel I in Kombination mit einem Fibrat, wie z.B. Fenofibrat, Clofibrat oder Bezafibrat, verabreicht.

Bei einer Ausführungsform der Erfindung wird die Verbindung der Formel I in Kombination mit einem MTP-Inhibitor, wie z.B. Implitapide, BMS-201038, R-103757 oder solchen wie in WO2005085226 beschrieben, verabreicht.

Bei einer Ausführungsform der Erfindung wird die Verbindung der Formel I in Kombination mit einem CETP-Inhibitor, wie z.B. Torcetrapib oder JTT-705, verabreicht.

Bei einer Ausführungsform der Erfindung wird die Verbindung der Formel I in Kombination mit einem Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9 beschrieben, verabreicht.

Bei einer Ausführungsform der Erfindung wird die Verbindung der Formel I in Kombination mit einem polymeren Gallensäureadsorber, wie z.B. Cholestyramin oder Colesevelam, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586 oder solchen wie in WO2005097738 beschrieben, verabreicht.

Bei einer Ausführungsform wird die Verbindung der Formel I in Kombination mit Omacor® (Omega-3-Fettsäuren; hochkonzentrierte Ethylester der Eicosapentaensäure und der Docosahexaensäure) verabreicht.

Bei einer Ausführungsform der Erfindung wird die Verbindung der Formel I in Kombination mit einem ACAT-Inhibitor, wie z.B. Avasimibe, verabreicht.

Bei einer Ausführungsform der Erfindung wird die Verbindung der Formel I in Kombination mit einem Antioxidans, wie z.B. OPC-14117, Probucol, Tocopherol, Ascorbinsäure, ß-Caroten oder Selen, verabreicht.

Bei einer Ausführungsform der Erfindung wird die Verbindung der Formel I in Kombination mit einem Vitamin, wie z. B. Vitamin B6 oder Vitamin B12, verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform der Erfindung wird die Verbindung der Formel I in Kombination mit einem Squalen Synthetase Inhibitor, wie z.B. BMS-188494 oder wie in WO2005077907 beschrieben, verabreicht.

Bei einer Ausführungsform der Erfindung wird die Verbindung der Formel I in Kombination mit einem Lipoprotein(a) Antagonist, wie z.B. Gemcabene (Cl-1027), verabreicht.

Bei einer Ausführungsform der Erfindung wird die Verbindung der Formel I in Kombination mit einem HM74A Rezeptor Agonisten, wie z.B. Nicotinsäure, verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit Insulin verabreicht.

Bei einer Ausführungsform der Erfindung wird die Verbindung der Formel I in Kombination mit einem Sulfonylharnstoff, wie z.B. Tolbutamid, Glibenclamid, Glipizid oder Glimepirid, verabreicht.

Bei einer Ausführungsform der Erfindung wird die Verbindung der Formel I in Kombination mit einem Biguanid, wie z.B. Metformin, verabreicht.

Bei wieder einer Ausführungsform der Erfindung wird die Verbindung der Formel I in Kombination mit einem Meglitinid, wie z.B. Repaglinide oder Nateglinid, verabreicht.

Bei einer Ausführungsform der Erfindung wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform der Erfindung wird die Verbindung der Formel I in Kombination mit einem α-Glukosidase-Inhibitor, wie z.B. Miglitol oder Acarbose, verabreicht.

Bei einer Ausführungsform der Erfindung wird die Verbindung der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform der Erfindung wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc., verabreicht.

Bei einer Ausführungsform der Erfindung wird die Verbindung der Formel I in Kombination mit einem Hemmstoff der Glykogen Phosphorylase, wie z.B. PSN-357 oder FR-258900 oder solchen wie in WO2003084922, WO2004007455, WO2005073229-31 oder WO2005067932 beschrieben, verabreicht.

Bei einer Ausführungsform der Erfindung wird die Verbindung der Formel I in Kombination mit Glukagon-Rezeptor-Antagonisten, wie z.B. A-770077, NNC-25-2504 oder wie in WO2004100875 oder WO2005065680 beschrieben, verabreicht.

Bei einer Ausführungsform der Erfindung wird die Verbindung der Formel I in Kombination mit Aktivatoren der Glukokinase, wie z. B. RO-4389620, LY-2121260 (WO2004063179), PSN-105, PSN-110, GKA-50oder solchen wie sie z. B. von Prosidion in WO2004072031, WO2004072066, WO 05103021 oder WO 06016178, von Roche in WO 00058293, WO 00183465, WO 00183478, WO 00185706, WO 00185707, WO 01044216, GB 02385328, WO 02008209, WO 02014312, WO 0246173, WO 0248106, DE 10259786, WO 03095438, US 04067939 oder WO 04052869, von Novo Nordisk in EP 1532980, WO 03055482, WO 04002481, WO 05049019, WO 05066145 oder WO 05123132, von Merck/Banyu in WO 03080585, WO03097824, WO 04081001, WO 05063738 oder WO 05090332, von Eli Lilly in WO 04063194, oder von Astra Zeneca in WO 01020327, WO 03000262, WO 03000267, WO 03015774, WO 04045614, WO 04046139, WO 05044801, WO 05054200, WO 05054233, WO 05056530, WO 05080359, WO 05080360 oder WO 05121110 beschrieben sind, verabreicht.

Bei einer Ausführungsform der Erfindung wird die Verbindung der Formel I in Kombination mit einem Inhibitor der Glukoneogenese, wie z. B. FR-225654, verabreicht.

Bei einer Ausführungsform der Erfindung wird die Verbindung der Formel I in Kombination mit Inhibitoren der Fructose-1,6-bisphosphatase (FBPase), wie z.B. CS-917, verabreicht.

Bei einer Ausführungsform der Erfindung wird die Verbindung der Formel I in Kombination mit Modulatoren des Glukosetransporters-4 (GLUT4), wie z.B. KST-48 (D.-O. Lee et al.: Arzneim.-Forsch. Drug Res. 54 (12), 835 (2004)), verabreicht.

Bei einer Ausführungsform der Erfindung wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform der Erfindung wird die Verbindung der Formel I in Kombination mit Inhibitoren der Dipeptidylpeptidase-IV (DPP-IV), wie z. B. Vildagliptin (LAF-237), Sitagliptin (MK-0431), Saxagliptin ((BMS-477118), GSK-823093, PSN-9301, SYR-322, SYR-619, TA-6666, TS-021, GRC-8200, GW-825964X oder wie sie in WO2003074500, WO2003106456, WO200450658, WO2005058901, WO2005012312, WO2005/012308, PCT/EP2005/007821, PCT/EP2005/008005, PCT/EP2005/008002, PCT/EP2005/008004, PCT/EP2005/008283, DE 10 2005 012874.2 oder DE 10 2005 012873.4 beschrieben sind, verabreicht.

Bei einer Ausführungsform der Erfindung wird die Verbindung der Formel I in Kombination mit Hemmstoffen der 11-beta-Hydroxysteroid-Dehydrogenase-1 (11β-HSD1), wie z. B. BVT-2733 oder solche, wie sie z. B. in WO200190090-94, WO200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004065351, WO2004089367, WO2004089380, WO2004089470-71, WO2004089896, WO2005016877 oder WO2005097759 beschrieben sind, verabreicht.

Bei einer Ausführungsform der Erfindung wird die Verbindung der Formel I in Kombination mit Inhibitoren der Protein-Tyrosin-Phosphatase-1 B (PTP1 B), wie sie z. B. in WO200119830-31, WO200117516, WO2004506446, WO2005012295, PCT/EP2005/005311, PCT/EP2005/005321, PCT/EP2005/007151, PCT/EP2005/01294 oder DE 10 2004 060542.4 beschrieben sind, verabreicht.

Bei einer Ausführungsform der Erfindung wird die Verbindung der Formel I in Kombination mit Modulatoren des natrium-abhängigen Glukosetransporters 1 oder 2 (SGLT1, SGLT2), wie z.B. KGA-2727, T-1095 und SGL-0010 oder wie sie z. B. in WO2004007517, WO200452903, WO200452902, WO2005121161, WO2005085237, JP2004359630 oder von A. L. Handlon in Expert Opin. Ther. Patents (2005) 15(11), 1531-1540 beschrieben sind, verabreicht.

Bei einer Ausführungsform der Erfindung wird die Verbindung der Formel I in Kombination mit Inhibitoren der hormon-sensitiven Lipase (HSL), wie z. B. in in WO01/17981, WO01/66531, WO2004035550, WO2005073199 oder WO03/051842 beschrieben, verabreicht.

Bei einer Ausführungsform der Erfindung wird die Verbindung der Formel I in Kombination mit Hemmstoffen der Acetyl-CoA Carboxylase (ACC) wie z. B. solchen wie in WO199946262, WO200372197, WO2003072197 oder WO2005044814 beschrieben, verabreicht.

Bei einer Ausführungsform der Erfindung wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform der Erfindung wird die Verbindung der Formel I in Kombination mit einem Inhibitor der Glykogen Synthase Kinase-3 beta (GSK-3 beta), wie z. B. in US2005222220, WO2004046117, WO2005085230, WO2005111018, WO2003078403, WO2004022544, WO2003106410, WO2005058908, US2005038023, WO2005009997, US2005026984, WO2005000836, WO2004106343, EP1460075, WO2004014910, WO2003076442, WO2005087727 oder WO2004046117 beschrieben, verabreicht.

Bei einer Ausführungsform der Erfindung wird die Verbindung der Formel I in Kombination mit einem Inhibitor der Protein Kinase C beta (PKC beta), wie z. B. Ruboxistaurin, verabreicht.

Bei einer Ausführungsform der Erfindung wird die Verbindung der Formel I in Kombination mit einem Endothelin-A-Rezeptor Antagonisten, wie z. B. Avosentan (SPP-301), verabreicht.

Bei einer Ausführungsform der Erfindung wird die Verbindung der Formel I in Kombination mit Inhibitoren der "I-kappaB Kinase" (IKK Inhibitoren), wie sie z. B. in WO2001000610, WO2001030774, WO2004022553 oder WO2005097129 beschrieben sind, verabreicht.

Bei einer Ausführungsform der Erfindung wird die Verbindung der Formel I in Kombination mit Modulatoren des Glukocorticoidrezeptors, wie sie z. B. in WO2005090336 beschrieben sind, verabreicht.

Bei einer weiteren Ausführungsform der Erfindung wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558); NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A);
Peptid YY 3-36 (PYY3-36) oder analoge Verbindungen, wie z. B. CJC-1682 (PYY3-36 konjugiert mit humanem Serum Albumin über Cys34), CJC-1643 (Derivat des PYY3-36, welches sich in vivo an Serum Albumin konjugiert) oder solche, wie sie in WO2005080424 beschrieben sind;
Cannabinoid Rezeptor 1 Antagonisten, wie z.B. Rimonabant, SR147778 oder solche wie sie in z. B. EP 0656354, WO 00/15609, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456, WO2005063761-62, WO2005061509 oder WO2005077897 beschrieben sind;
MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chlorophenyl)-2-oxo-ethyl]-amid; (WO 01/91752)) oder LB53280, LB53279, LB53278 oder THIQ, MB243, RY764, CHIR-785, PT-141 oder solche wie sie in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO004005324, WO2004037797, WO2005042516, WO2005040109, WO2005030797, US20040224901, WO200501921, WO200509184, WO2005000339, EP1460069, WO2005047253, WO2005047251, EP1538159, WO2004072076, WO2004072077 oder WO2006024390 beschrieben sind;
Orexin-Rezeptor Antagonisten (z.B. 1-(2-Methyl-benzoxazol-6-yl)-3-[1,5]naphthyridin-4-yl-harnstoff Hydrochlorid (SB-334867-A) oder solche, wie sie z. B. in WO200196302, WO200185693, WO2004085403 oder WO2005075458 beschrieben sind);
Histamin H3 Rezeptor Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO200064884, WO2005082893 beschrieben sind); CRF-Antagonisten (z.B. [2-Methyl-9-(2,4,6-trimethyl-phenyl)-9H-1,3,9-triaza-fluoren-4-yl]-dipropyl-amin (WO 00/66585));
CRF BP-Antagonisten (z.B. Urocortin);
Urocortin-Agonisten;
β3-Agonisten (wie z.B. 1-(4-Chloro-3-methanesulfonylmethyl-phenyl)-2-[2-(2,3-dimethyl-1 H-indol-6-yloxy)-ethylamino]-ethanol Hydrochlorid (WO 01/83451));
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71, GW-803430 oder solche Verbindungen, wie sie in WO2003/15769, WO2005085200, WO2005019240, WO2004011438, WO2004012648, WO2003015769, WO2004072025, WO2005070898, WO2005070925, WO2006018280, WO2006018279, WO2004039780, WO2003033476, WO2002006245, WO2002002744, WO2003004027 oder FR2868780 beschrieben sind);
CCK-A Agonisten (wie z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525), SR-146131 (WO 0244150) oder SSR-125180);
Serotonin-Wiederaufnahme-Inhibitoren (z.B. Dexfenfluramine);
gemischte Serotonin- und noradrenerge Verbindungen (z.B. WO 00/71549);
5-HT-Rezeptor Agonisten z.B. 1-(3-Ethyl-benzofuran-7-yl)-piperazin Oxalsäuresalz (WO 01/09111);
5-HT2C Rezeptor Agonisten (wie z.B. APD-356, BVT-933 oder solche, wie sie in WO200077010, WO20077001-02, WO2005019180, WO2003064423, WO200242304 oder WO2005082859 beschrieben sind);
5-HT6 Rezeptor Antagonisten, wie sie z.B. in WO2005058858 beschrieben sind;
Bombesin-Rezeptor Agonisten (BRS-3 Agonisten);
Galanin-Rezeptor Antagonisten;
Wachstumshormon (z.B. humanes Wachstumshormon oder AOD-9604);
Wachstumshormon freisetzende Verbindungen (6-Benzyloxy-1-(2-diisopropylaminoethylcarbamoyl)-3,4-dihydro-1 H-isochinolin-2-carbonsäuretertiärbutylester (WO 01/85695));
Growth Hormone Secretagogue Receptor Antagonisten (Ghrelin Antagonisten) wie z. B. A-778193 oder solche, wie sie in WO2005030734 beschrieben sind;
TRH-Agonisten (siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
DA-Agonisten (Bromocriptin oder Doprexin);
Lipase/Amylase-Inhibitoren (wie sie z.B. in WO 00/40569 beschrieben sind);
Inhibitoren der Diacylglycerol O-Acyltransferasen (DGATs) wie z. B. in US2004/0224997, WO2004094618, WO200058491, WO2005044250, WO2005072740, JP2005206492 oder WO2005013907 beschrieben;
Inhibitoren der Fettsäuresynthase (FAS) wie z.B. C75 oder solche, wie in WO2004005277 beschrieben;
Oxyntomodulin;
Oleoyl-Estron
oder Agonisten des Schilddrüsenhormonrezeptors (thyroid hormone receptor agonists) wie z. B: KB-2115 oder solche, wie in WO20058279, WO200172692, WO200194293, WO2003084915, WO2004018421 oder WO2005092316 beschrieben, verabreicht.

Bei einer Ausführungsform der Erfindung ist der weitere Wirkstoff Leptin;
siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform der Erfindung ist der weitere Wirkstoff Dexamphetamin oder Amphetamin.

Bei einer Ausführungsform der Erfindung ist der weitere Wirkstoff Fenfluramin oder Dexfenfluramin.

Bei noch einer Ausführungsform der Erfindung ist der weitere Wirkstoff Sibutramin.

Bei einer Ausführungsform der Erfindung ist der weitere Wirkstoff Mazindol oder Phentermin.

Bei einer Ausführungsform der Erfindung wird die Verbindung der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6). Caromax ist ein Carob enthaltendes Produkt der Fa. Nutrinova, Nutrition Specialties & Food Ingredients GmbH, Industriepark Höchst, 65926 Frankfurt / Main) verabreicht. Die Kombination mit Caromax^{®} kann in einer Zubereitung erfolgen oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Bei einer Ausführungsform der Erfindung wird die Verbindung der Formel I in Kombination mit PDE Inhibitoren (Phosphodiesterase), wie sie z. B. in WO2003/077949 oder WO2005012485 beschrieben sind, verabreicht.

Bei einer Ausführungsform der Erfindung wird die Verbindung der Formel I in Kombination mit NAR-1 (Nicotinic Acid Receptor) Agonisten, wie sie z. B. in WO2004094429 beschrieben sind, verabreicht.

Bei einer Ausführungsform der Erfindung wird die Verbindung der Formel I in Kombination mit CB2 (Cannabinoid Receptor) Agonisten, wie sie z. B. in US2005/143448 beschrieben sind, verabreicht.

Bei einer Ausführungsform der Erfindung wird die Verbindung der Formel I in Kombination mit Histamine-1-Agonisten, wie sie z. B. in WO2005101979 beschrieben sind, verabreicht.

Bei einer Ausführungsform der Erfindung wird die Verbindung der Formel I in Kombination mit Bupropion, wie in WO2006017504 beschrieben, verabreicht.

Bei einer Ausführungsform der Erfindung wird die Verbindung der Formel I in Kombination mit Opioid Antagonisten, wie sie z. B. in WO2005107806 oder WO2004094429 beschrieben sind, verabreicht.

Bei einer Ausführungsform der Erfindung wird die Verbindung der Formel I in Kombination mit Neutralen Endopeptidase Inhibitoren , wie sie z. B. in WO200202513, WO2002/06492, WO 2002040008, WO2002040022 oder WO2002047670 beschrieben sind, verabreicht.

Bei einer Ausführungsform der Erfindung wird die Verbindung der Formel I in Kombination mit NPY Inhibitoren (Neuropeptid Y), wie sie z. B. in WO2002047670 beschrieben sind, verabreicht.

Bei einer Ausführungsform der Erfindung wird die Verbindung der Formel I in Kombination mit Natrium/Wasserstoff Austausch Inhibitoren, wie sie z. B. in WO2003092694 beschrieben sind, verabreicht.

Bei einer Ausführungsform der Erfindung wird die Verbindung der Formel I in Kombination mit Modulatoren des Glukocorticoidrezeptors, wie sie z. B. in WO2005090336 beschrieben sind, verabreicht.

Bei einer Ausführungsform der Erfindung wird die Verbindung der Formel I in Kombination mit Nikotin Rezeptor Agonisten, wie sie z. B. in WO2004094429 beschrieben sind, verabreicht.

Bei einer Ausführungsform der Erfindung wird die Verbindung der Formel I in Kombination mit NRIs (Norepinephrine reuptake inhibitors), wie sie z. B. in WO2002053140 beschrieben sind, verabreicht.

Bei einer Ausführungsform der Erfindung wird die Verbindung der Formel I in Kombination mit MOA (E-beta-methoxyacrylate), wie z.B. Segeline oder wie sie z. B. in WO2002053140 beschrieben sind, verabreicht.

Bei einer Ausführungsform der Erfindung wird die Verbindung der Formel I in Kombination mit antithrombotischen Wirkstoffen, wie z. B. Clopidrogel, verabreicht.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Zu den vorstehend genannten Entwicklungskodes werden nachfolgend teilweise die Formeln aufgeführt.

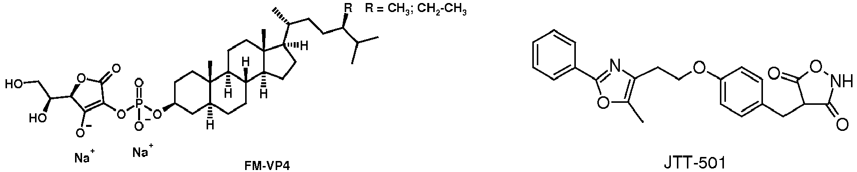

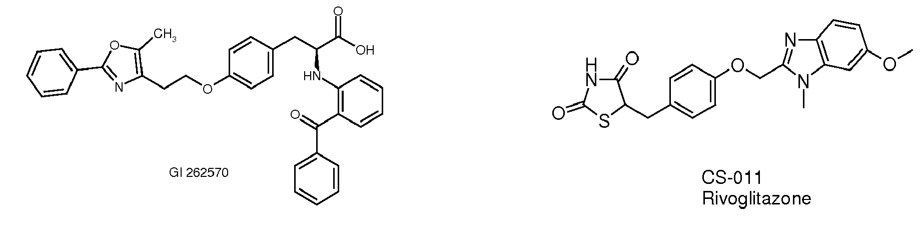

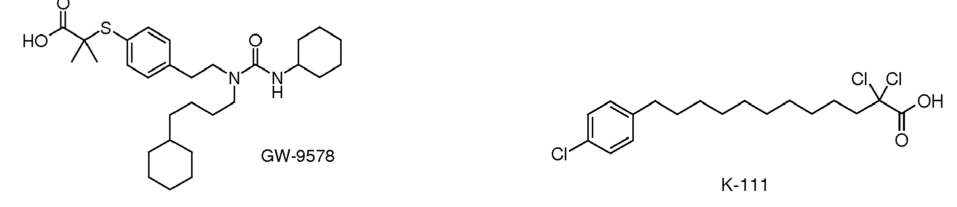

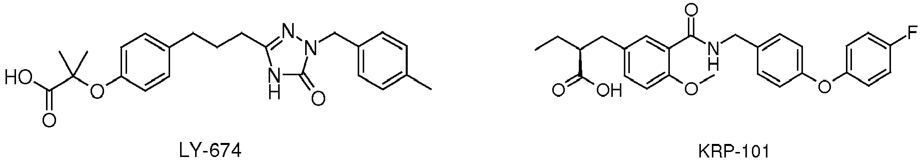

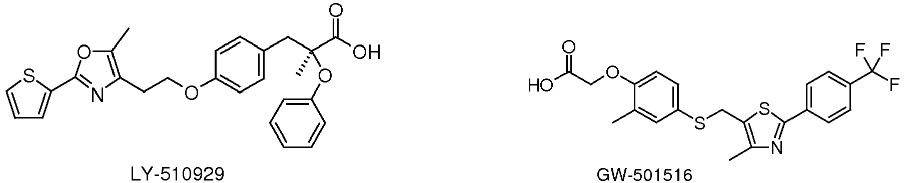

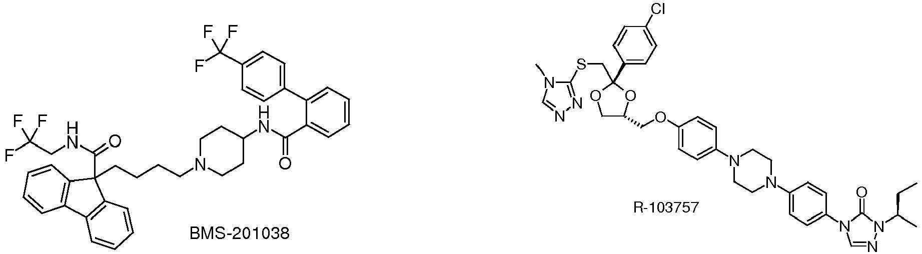

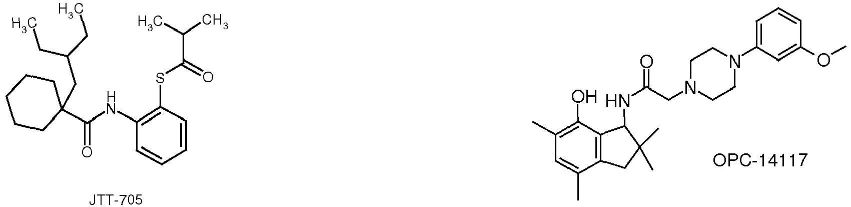

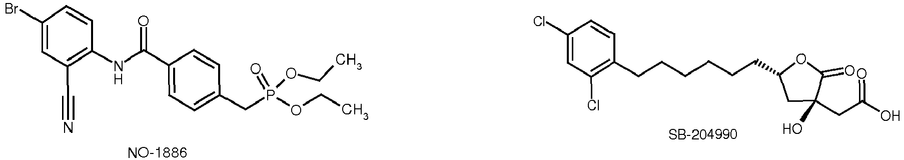

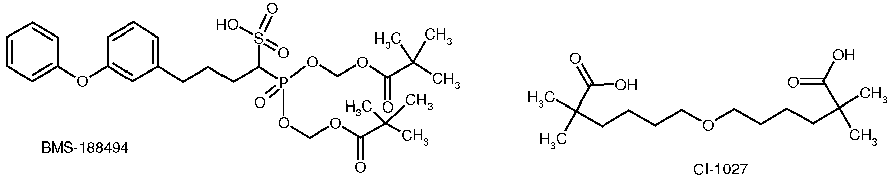

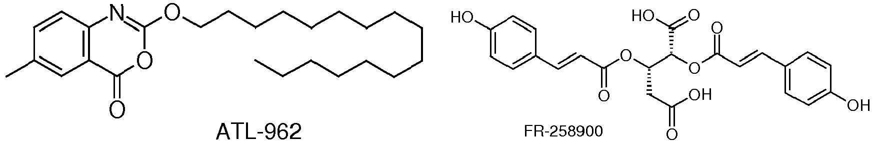

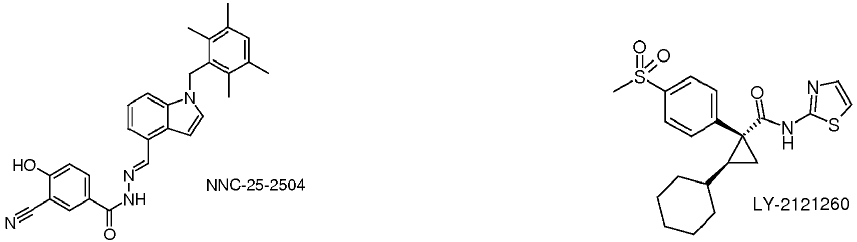

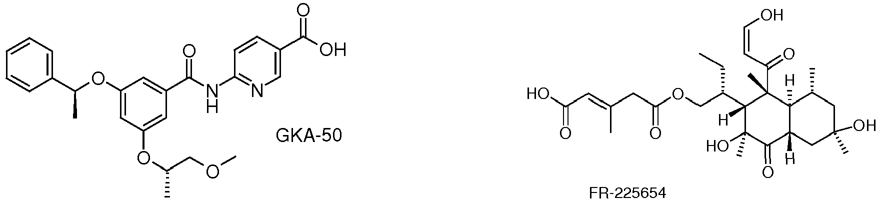

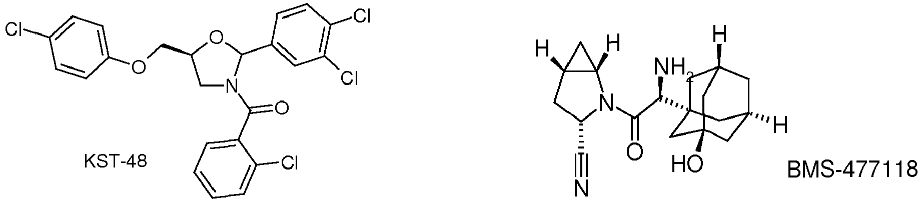

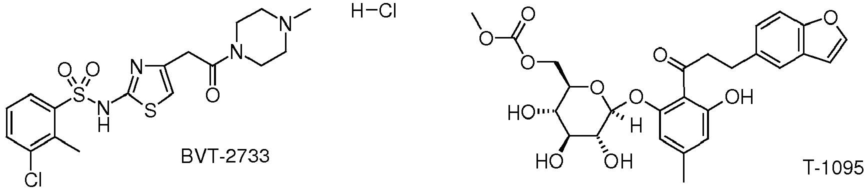

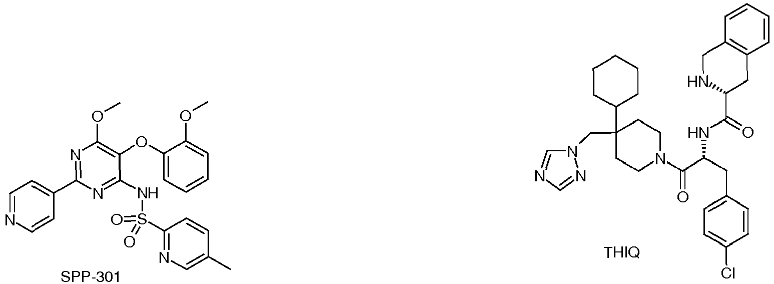

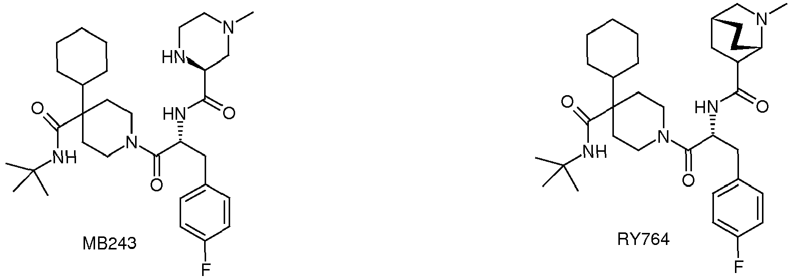

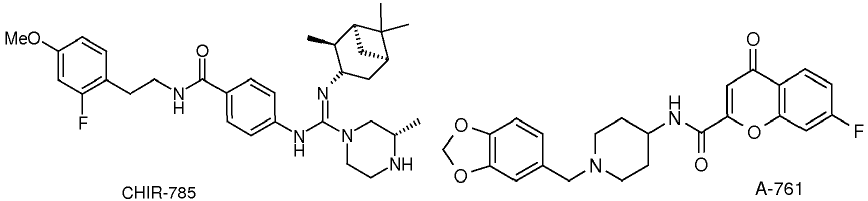

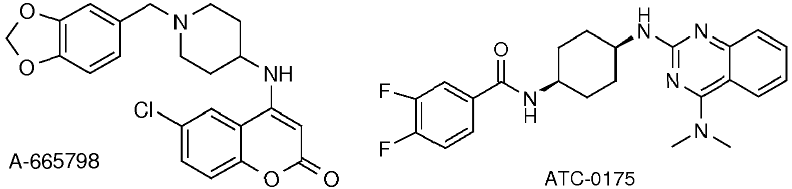

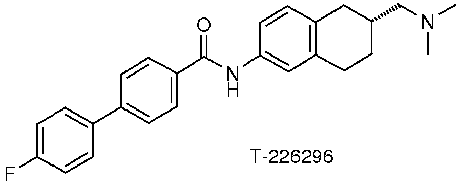

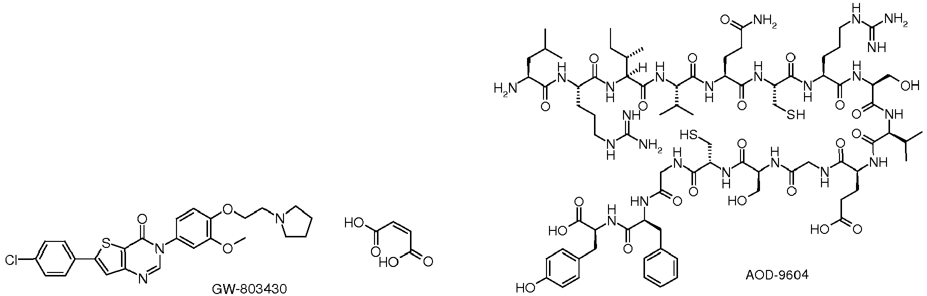

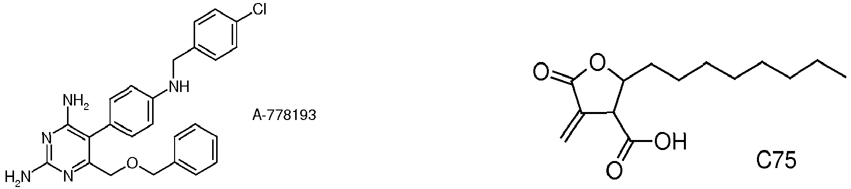

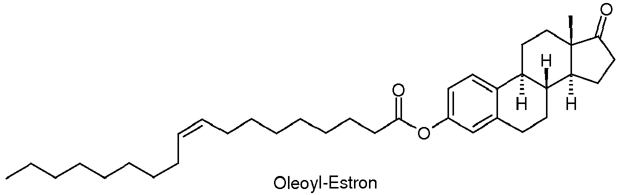

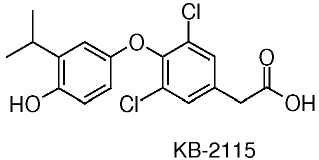

Die Wirksamkeit der erfindungsgemäßen Verbindungen der Formel I wurde an folgenden Enzymtestsystem geprüft:

### Test auf Hemmung der EL:

EL wird als sekretorisches Protein von rekombinanten Zell-Linien (CHO, HEK293) in hoher Konzentration in Zellkulturmedium abgegeben (konditioniertes Medium). Dieses wird nach Aufkonzentration als Enzymlösung eingesetzt.

### Assay auf EL-Aktivität

Zur Charakterisierung der enzymatischen Aktivität von endothelialer Lipase und der Wirkung von Inhibitoren wird das Phospholipase-spezifische Substrat 1,2-bis-(4,4-difluoro-5,7-dimethyl-4-bora-3a,4a-diaza-*s*-indacene-3-undecanoyl)-*sn*-glycero-3-phosphocholine, (Hersteller Molecular Probes) verwendet. Durch Hydrolyse der A1 Esterbindung dieses Phospholipids durch das Enzym wird eine mit dem Fluoreszenzfarbstoff Bodipy markierte Fettsäure freigesetzt, der nach Trennung durch Dünnschichtchromatographie auf einer HPTLC-Platte (Kieselgel 60, Merck ) oder direkt im Reaktionsgefäß durch Messen der Fluoreszenz nachgewiesen werden kann. Zur Herstellung der Substratlösung werden 100 µg 1,2-bis-(4,4-difluoro-5,7-dimethyl-4-bora-3a,4a-diaza-s-indacene-3-undecanoyl)-sn-glycero-3-phospho-choline (Hersteller Molecular Probes) in 100µl DMSO gelöst und in 2,4 mg Tripalmitin (Sigma) in 393 µl Chloroform aufgenommen, welches 20mg/ ml DOP - Cholin (1,2-Dioleoyl-sn-glycero-3-phosphocholin) enthält. 39,3µl diesesLipidgemisches werden in ein frisches Reaktionsgefäß überführt und das Lösungsmittel abgedampft. Das Lipidgemisch wird in 4 ml 200 mM TRIS-HCl, 150 mM Natriumchlorid, pH = 7,4 durch zweimaliges Sonifizieren gelöst. Die anschließende Enzymreaktion erfolgt für 90 Minuten bei 37°C. Hierzu werden 20 µl der Substratlösung mit 2 µl Inhibitor entsprechender Konzentration (gelöst in 10% DMSO, zur Kontrolle wird 10%ige DMSO-Lösung verwendet) und 2 µl Enzymlösung (konditioniertes Medium) inkubiert. Im Anschluß werden 4 µl des Testansatzes auf eine HPTLC-Platte (Kieselgel 60, Merck) aufgetragen und der freigesetzte Fluoreszenzfarbstoff zum Nachweis mit einem Fließmittel (Diethylether : Petroleumbenzin : Essigsäure [78:22:1]) getrennt. Nach dem Abdampfen des Fließmittels wird die Platte in einen Fluoreszenz-Scanner eingelesen. Als Maß für die Enzymaktivität ist eine gesteigerte Freisetzung des Fluoreszenzfarbstoffes in der ungehemmten Reaktion zu beobachten.

In Abhängigkeit der verwendeten Inhibitorkonzentration ergibt sich eine Reduktion der enzymatischen Aktivität, die Inhibitiorkonzentration, bei welcher eine halbmaximale Enzymaktivität beobachtet wird, wird als IC₅₀ bezeichnet.

| Beispiel | IC₅₀ [µM] EL |
|---|---|
| 6 | 0,028 |
| 7 | 0,9 |
| 8 | 3,2 |
| 16 | 0,004 |
| 18 | 14,1 |
| 25 | 0,009 |
| 26 | 0,035 |
| 39 | 0,001 |
| 40 | 0,053 |

### Andere Prüfmodelle

Anhand verschiedener Prüfmodelle kann die Eignung der erfindungsgemäßen Verbindungen als pharmazeutischer Wirkstoff getestet werden. Im Folgenden werden beispielhaft Beschreibungen solcher Prüfmodelle gegeben.

### Löslichkeiten in wässrigen Systemen

Ausreichende Löslichkeit einer Substanz in wässrigen Lösungsmittelsystemen ist eine wichtige Vorraussetzung für eine (reproduzierbare) pharmakologische Wirkung. Löslichkeiten in wässrigen Systemen können nach verschiedenen Verfahren bestimmt werden. Geeignet sind z. B. Fällungsverfahren aus Lösungen ("kinetische Löslichkeit") und Verfahren, die die Auflösung einer festen Probe bis zur Gleichgewichtseinstellung untersuchen ("thermodynamische Löslichkeit").

### a) Kinetische Löslichkeit

Auf einer 96-well Mikrotiterplatte wird eine DMSO-Lösung der Testverbindung (2,5 mM; 0,5 µL) zu 200 µL einer wässrigen Testlösung (z. B. Phosphate buffered Saline, 10x, 1 M, Sigma eingestellt auf 10 mM, pH 7,4) pipettiert und die Trübung bei der so erhaltenen theoretischen Konzentration der Testverbindung von 6,25 µM mittels eines Nephelometers (z. B. Nephelostar Galaxy, BMG Labtech) gemessen. Danach wird die Konzentration der Testverbindung in der wässrigen Testlösung durch Zugabe von weiterer DMSO-Lösung (2,5 mM; 0,5 µL) auf theoretisch 12,5 µM erhöht und die Trübungsmessung erneut durchgeführt. Weitere Zugaben von DMSO-Lösungen (1 µL, 2,5 mM; 0,5 µL, 10 mM; dann 9-mal 1 µL, 10 mM ergebend theoretische Konzentrationen von 25 µM, 50 µM, 100 µM, 150 µM, 200 µM, 250 µM, 300 µM, 350 µM, 400 µM, 450 µM und 500 µM) mit zwischenzeitlicher Trübungsmessung komplettieren den Messprozess.
Auswertung: Die Trübungswerte des Nephelometers werden gegen die theoretische Konzentration der Testverbindung in der wässrigen Testlösung aufgetragen. Sobald bei einer theoretischen Konzentration eine signifikante Trübung detektiert wird (z. B. 5-fach über dem Kontrollwert der wässrigen Testlösung), wird der darunter liegende Konzentrationswert als Löslichkeitsgrenze der Testverbindung in der Testlösung angegeben. Als maximal möglicher Messbereich ergeben sich damit die Werte <6,25 µM, 6,25 - 500 µM und >500 µM.

Bevorzugte erfindungsgemäße Verbindungen zeigen eine kinetische Löslichkeit im Phosphatpuffer (pH 7,4) von mindestens 12,5 µM; bevorzugter von mindestens 50 µM und noch stärker bevorzugt von mindestens 250 µM.

### b) Thermodynamische Löslichkeit

Mittels HPLC-UV-Messung einer Verdünnungsreihe der Testverbindung in DMSO (500 µM, 100 µM, 50 µM, 10 µM und 1 µM) wird die integrierte UV-Absorption in einer Kalibiergeraden linear mit der Konzentration korreliert. Die Testverbindung (500 µg) wird zusammen mit der wässrigen Testlösung (250 µL) in einem geschlossenen Gefäß (Fassungsvolumen: 1,5 mL) für 16 Stunden geschüttelt (Eppendorf Thermoschüttler, 1400 rpm, 25°C, Abdeckung als Lichtschutz). Anschließend wird die Probe bei maximaler Drehzahl zentrifugiert und der Überstand abschließend noch filtriert. Eine Probe des filtrierten Überstandes wird direkt mittels HPLC-UV-Messung (siehe oben) analysiert. Eine weitere Probe wird nach Verdünnen (1 Volumenteil Überstand, 39 Volumenteile Testlösung) analysiert.
Auswertung: Anhand der erstellten Kalibiergeraden wird aus den erhaltenen integrierten UV-Absorptionen der Überstandsproben die Konzentration der Testverbindung im unverdünnten Überstand berechnet und als Löslichkeit der Testverbindung in der jeweiligen wässrigen Testlösung angegeben.
Beispiele für wässrige Testlösungen sind entsalztes Wasser oder wässrige Phosphatpuffer mit verschiedenen pH-Werten (z. B. pH 1,2; pH 4,0; pH 6,8; pH 7,4; pH 9,0), die nach Standardverfahren aus der kommerziellen Lösung (Phosphate buffered saline, 10x, Sigma) hergestellt werden können durch Verdünnen bzw. Einstellen mit Phosphorsäure oder Natronlauge.
Bevorzugte erfindungsgemäße Verbindungen zeigen eine Löslichkeit im Phosphatpuffer (pH 7,4) von mindestens 12,5 µM; bevorzugter von mindestens 50 µM und noch stärker bevorzugt von mindestens 250 µM.

### Metabolische Stabilität

Die metabolische Stabilität wird bestimmt durch Inkubation der Testverbindung (5 µM) bei 37°C mit microsomalen Leberfraktionen (1 mg/mL Protein mit 0,1 % w/v BSA; 1 mM NADPH, 0,5% DMSO). Die Analyse bei 0 und 20 Minuten Inkubationszeit erfolgt mittels LCMS/MS. Weitere Beschreibungen des Testsystems und Referenzen zur experimentellen Durchführung finden sich bei Plant, N.; Drug Discovery Today 2004, 9(7), 328-336 und Lau, Y.Y. et al.; Pharmaceutical Res. 2002, 19(11), 1606-1610.

### Verfahren zur Herstellung

Die Herstellung der erfindungsgemäßen Verbindungen der allgemeinen Formel I erfolgt nach an und für sich bekannten Methoden in zwei Schritten.

Substituierte Isoxazolone können durch Umsetzung von entsprechend substituierten Acetessigesterderivaten IIa mit Hydroxylamin hergestellt werden, wie beispielsweise in Bowden K., Crank C., Ross WJ., J. Chem. Soc. C 1968, 172-185 beschrieben. Die Acetessigesterderivate IIa sind zum einen als Handelsprodukte kommerziell erhältlich oder können aus Acetessigester nach an sich bekannten Methoden durch Alkyklierung hergestellt werden. 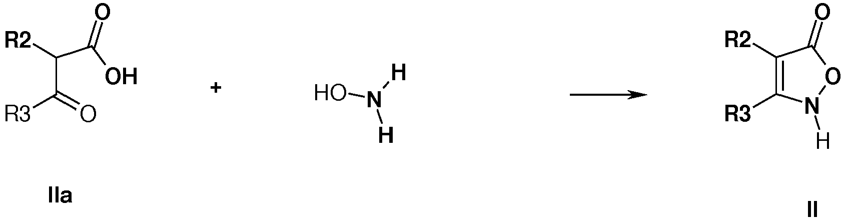 In einem weiteren Schritt werden die erfindungsgemäßen Verbindungen der Formel I hergestellt durch Acylierung der unsubstituierten, bzw. substituierten Isoxazolone II mit Carbamoylchloriden III (Methode A), oder in zwei Stufen durch Umsetzung von 3-Oxo-isoxazolen II mit Phosgen oder Äquivalenten wie Chlorcarbonsäuretrichlormethylester, Carbonsäureditrichlormethylester oder Chlorameisensäure-4-nitrophenylester und weiterer Umsetzung des erhaltenen Isoxazolone-carbonsäurederivats mit Aminen IV (Methode B), oder durch Umsetzung der Isoxazolone II mit den entsprechenden Isocyanaten V R1-N=C=O.

Da bei diesen Reaktionen in der Regel Säuren freigesetzt werden, empfiehlt es sich zur Beschleunigung Basen wie Pyridin, Triethylamin, Natronlauge oder Alkalicarbonate zu zusetzen. Die Reaktionen können in weiten Temperaturbereichen durchgeführt werden. In der Regel hat es sich als vorteilhaft herausgestellt, bei 0°C bis zum Siedepunkt des verwendeten Lösungsmittels zu arbeiten. Als Lösemittel kommen beispielsweise Methylenchlorid, THF, DMF, Toluol, Essigester, n-Heptan, Dioxan, Diethylether oder Pyridin zum Einsatz. Wenn unter wasserfreien Bedingungen gearbeitet wird, haben sich auch starke Basen wie Lithiumhydrid, Natriumhydrid oder Kalium-tert.-butylat in aprotischen Lösungsmitteln wie THF oder DMF bewährt.

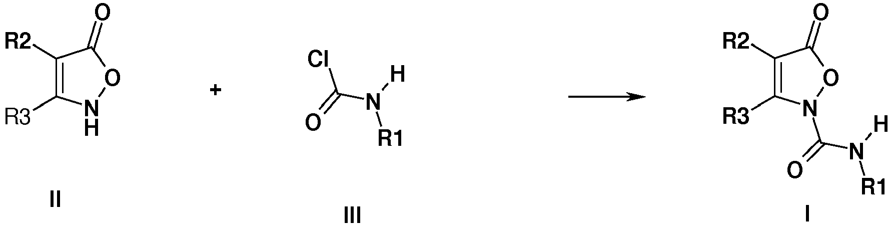

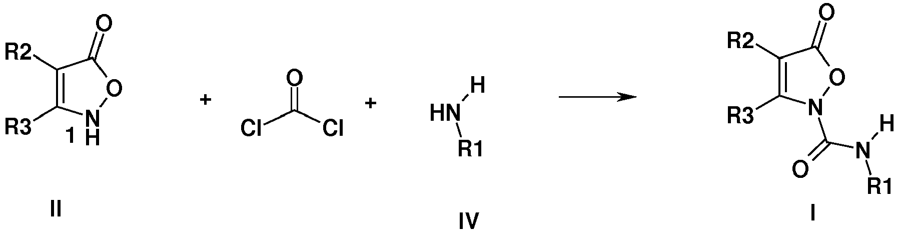

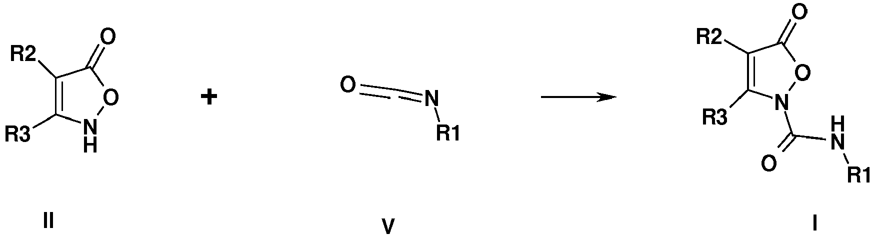

### Beispiele

### 3- Cyclopropyl-2H-isoxazol-5-on

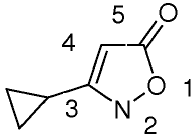

Eine Lösung von 3-Cyclopropyl-3-oxopropionsäuremethylester (4,8 g, 34 mmol) in Methanol (80 mL) wird mit Hydroxylaminhydrochlorid (2,6 g, 38 mmol) und Triethylamin (5,3 mL, 38 mmol) versetzt und der Ansatz für 2 h unter Rückfluß erhitzt. Das Lösungsmittel wird im Vakuum abdestilliert. Der Rückstand wird in EtOAc aufgenommen und über Kieselgel filtriert.
Ausbeute: 3,2 g (75,2%).

Isoxazolone, die in 3-Position unterschiedlich substituiert sind, oder in 4-Position zusätzliche Substituenten tragen wurden analog hergestellt. Hierbei kamen entweder käufliche substituierte Acetessigesterderivate zum Einsatz, oder wurden aus Acetessigester nach an sich bekannten Methoden durch Alkyklierung hergestellt.

### 5-Oxo-5H-isoxazol-2-carbonsäureamide

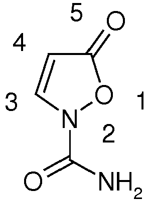

### 3-Cyclopropyl-5-oxo-5H-isoxazol-2-carbonsäure-thiophen-2-ylmethylamid

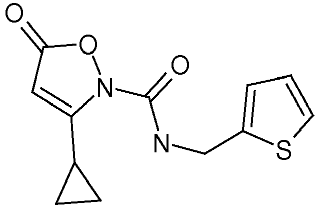

Eine Lösung von Phosgen in Toluol (20%, 1 mL, 2 mmol) wird mit THF (10 mL) verdünnt. Zu dieser Lösung gibt man eine Lösung von 3- Cyclopropyl-2H-isoxazol-5-on (125 mg, 1 mmol) in THF (5 mL), versetzt den Ansatz mit Triethylamin (140 µL, 1 mmol) und rührt für 8 h bei 25° C. Anschließend wird vom Niederschlag abfiltriert und das Filtrat im Vakuum eingeengt. Der Rückstand wird in THF (10 mL) aufgenommen und zu einer Lösung von 2-Thiophen-2 methylamin (113,2 mg, 1 mmol) in Pyridin (10 mL) zugegeben. Der Ansatz wird für 16 h bei 25° C gerührt. Anschließend wird das Lösungsmittel im Vakuum abdestilliert und der Rückstand durch HPLC gereinigt.
Ausbeute: 66 mg (25%)

Entsprechend wurden in 3- und 4-Position unterschiedlich substituierte Isoxazolone, durch Umsetzung mit unterschiedlichen Aminen zu den korrespondierenden 5-Oxo-5H-isoxazol-2-carbonsäureamiden umgesetzt.

Die nachfolgend aufgeführten Beispiele dienen zur Erläuterung der Erfindung, ohne diese jedoch einzuschränken.

### (mit * gekennzeichnete Verbindungen sind Referenzbeispiele)

| Bsp. | Chemische Struktur | R1 |
|---|---|---|
| 1* | 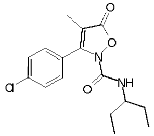 | 1-Ethylpropyl |
| 2 | 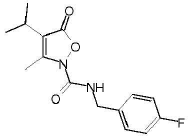 | 4-Fluorbenzyl |
| 3 | 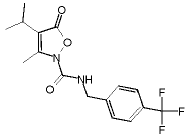 | 4-Trifluormethylbenzyl |
| 4* | 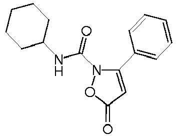 | Cyclohexyl |
| 5 | 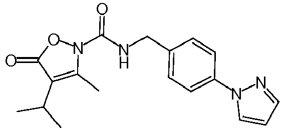 | 4-Pyrazol-1-yl-benzyl |
| 6 | 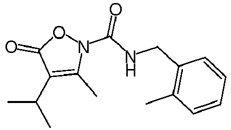 | 2-Methylbenzyl |
| 7 | 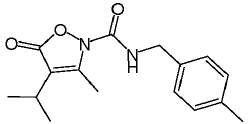 | 4-Methylbenzyl |
| 8 | 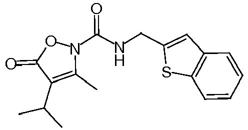 | Benzo-[b]-thiophen-2-yl-methyl |
| 9 | 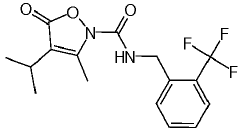 | 2-Trifluormethyl |
| 10* | 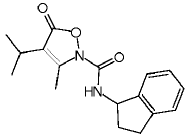 | Indan-1-yl |
| 11 | 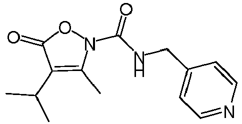 | Pyridin-4-yl-methyl |
| 12* | 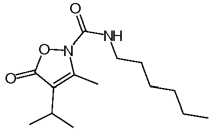 | n-Hexyl |
| 13* | 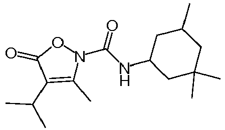 | 3,3,5 Trimethylcyclohexyl |
| 14 | 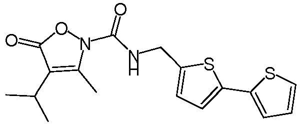 | [2,2']Bithiophenyl-5-methyl |
| 15 | 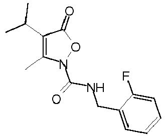 | 2-Fluorbenzyl |
| 16 | 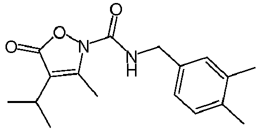 | 3,4-Dimethylbenzyl |
| 17 | 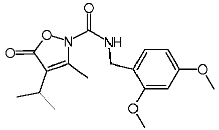 | 2,4-Dimethoxybenzyl |
| 18 | 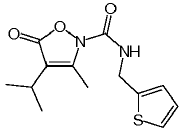 | Thiophen-2-ylmethyl |
| 19 | 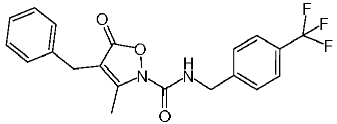 | 4-Trifluormethylbenzyl |
| 20 | 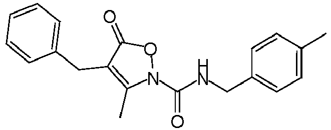 | 4-Methylbenzyl |
| 21 | 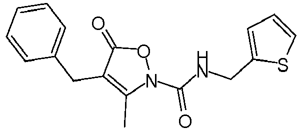 | Thiophen-2-ylmethyl |
| 22 | 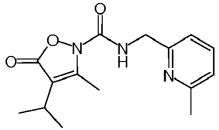 | 6-Methylpyridin-2-yl-methyl |
| 23 | 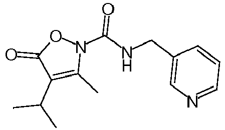 | Pyridin-3-yl-methyl |
| 24 | 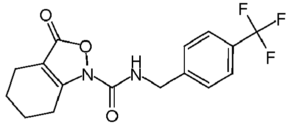 | 4-Trifluormethylbenzyl |
| 25 | 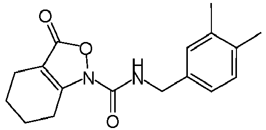 | 3,4-Dimethylbenzyl |
| 26 | 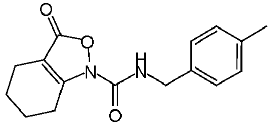 | 4-Methylbenzyl |
| 27 | 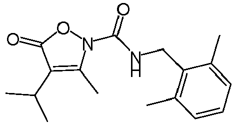 | 2,6-Diemthylbenzyl |
| 28 | 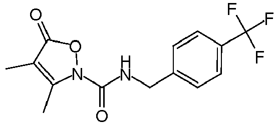 | 4-Trifluormethylbenzyl |
| 29 | 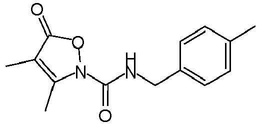 | 4-Methylbenzyl |
| 30 | 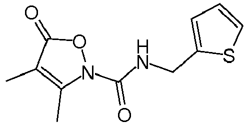 | Thiphen-2-yl-methyl |
| 31* | 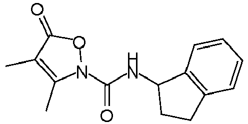 | Indan-1-yl |
| 32 | 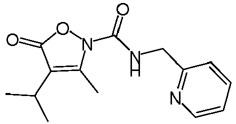 | Pyridin-2-yl-methyl |
| 33 | 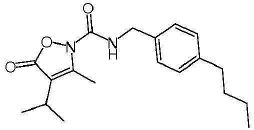 | 4-Butyl-benzyl |
| 34* | 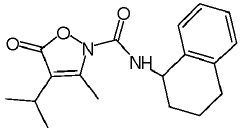 | 1,2,3,4-Tetrahydronaphtalen -1-yl |
| 35* | 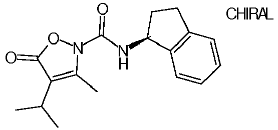 | (S)-Indan-1-yl |
| 36* | 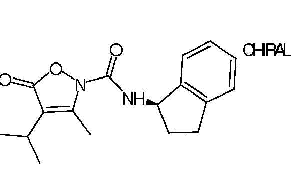 | (R)-Indan-1-yl |
| 37* | 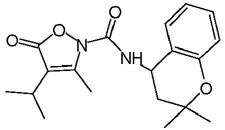 | 2,2-dimethyl-1-chroman-4-yl |
| 38* | 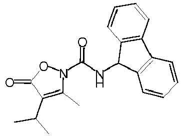 | 9H-fluoren-9-yl |
| 39 | 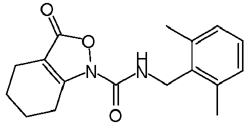 | 2,6-Dimethylbenzyl |
| 40 | 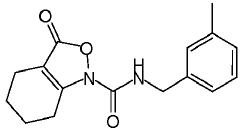 | 3-Methylbenzyl |
| 41 | 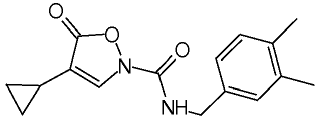 | 3,4-Dimethylbenzyl |
| 42 | 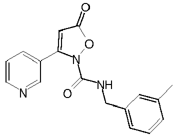 | 2-Methylbenzyl |
| 43 | 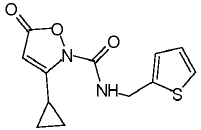 | Thiophen-2-ylmethyl |
| 44* | 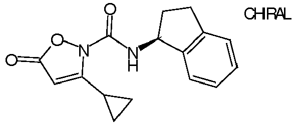 | (S)-Indan-1-yl |
| 45 | 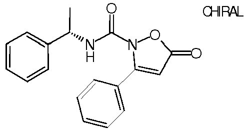 | (S)-1-Phenylethyl |
| 46 | 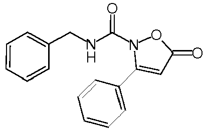 | Benzyl |
| 47* | 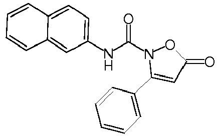 | 2-Naphthyl |
| 48 | 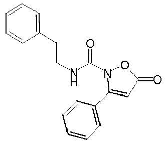 | Phenylethyl |
| 49 | 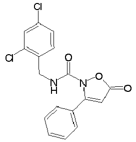 | 3,5-dichlorbenzyl |
| 50* | 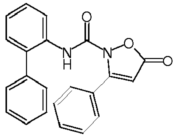 | Biphenyl-2-yl |
| 51* | 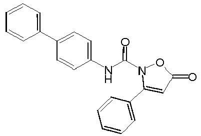 | Biphenyl-4-yl |
| 52 | 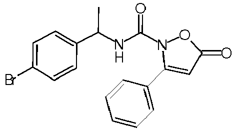 | 1-(4-bromophenyl)-ethyl |
| 53 | 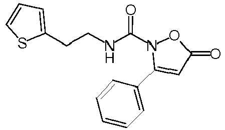 | 2-Thiophen-2yl-ethyl |
| 54 | 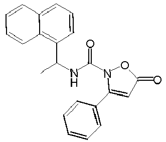 | 1-Naphthalen-1 ylethyl |
| 55 | 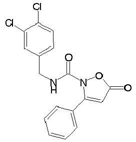 | 3,4-Dichlorophenyl |
| 56 | 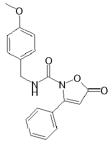 | 4-Methyloxybenzyl |
| 57* | 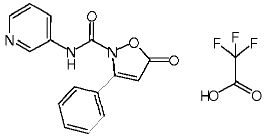 | Pyridin-3-yl- |
| 58* | 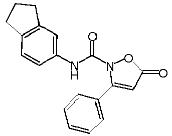 | Indan-5-yl |
| 59* | 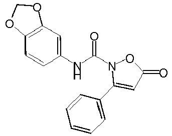 | benzo-1,3-dioxol-5-yl |
| 60* | 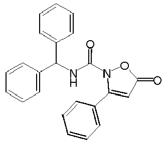 | 1,1-Diphenylmethyl |
| 61 | 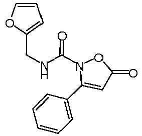 | Furan-2-ylmethyl |
| 62 | 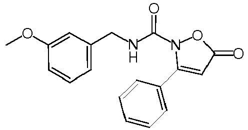 | 3-Methyloxybenzyl |
| 63* | 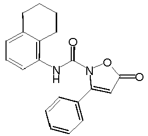 | 1,2,3,4-Tetrahydronaphtalen -1-yl |
| 64 | 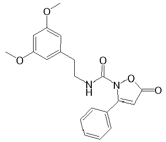 | 2-(3,5-Dimethyloxyphenyl)e thyl |
| 65 | 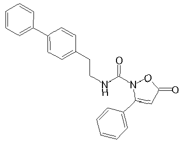 | 2-Biphenyl-4-yl-ethyl |
| 66* | 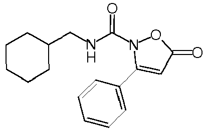 | Cyclohexylmethyl |
| 67 | 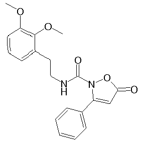 | 2-(2,3-dimethyloxyphenyl)e thyl |
| 68 | 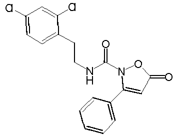 | 2,3,4Dichlorophenylethyl |
| 69 | 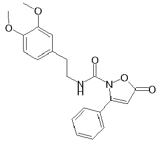 | 3,4-Dimethyloxyethylphe nyl |
| 70* | 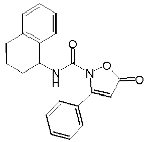 | 1,2,3,4-Tetrahydronaphtalen -1-yl |
| 71* | 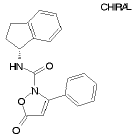 | (R)-Indan-1-yl |
| 72* | 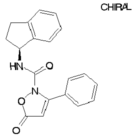 | (S)-Indan-1-yl |
| 73 | 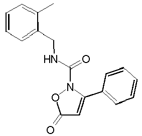 | 2-Methylbenzyl |
| 74 | 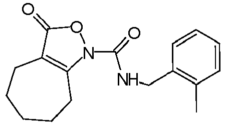 | 2-Methylbenzyl |
| 75 | 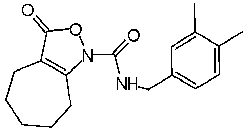 | 3,4-Dimethylbenzyl |
| 76 | 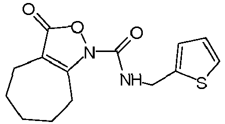 | Thiophen-2-yl-methyl |
| 77 | 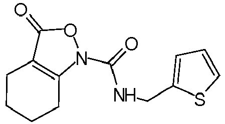 | Thiophen-2-yl-methyl |
| 78* | 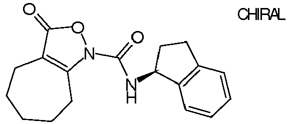 | (S)-Indan-1-yl |
| 79* | 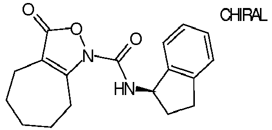 | (R)-Indan-1-yl |
| 80 | 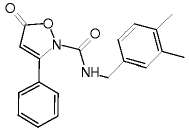 | 3,4-Dimethylbenzyl |

## Patentansprüche

1. Verbindung der allgemeinen Formel I 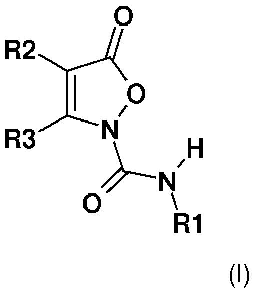 wobei bedeuten:
R1 Y-Aryl, Y-Heteroaryl, wobei Aryl oder Heteroaryl ein oder mehrfach durch, F, Cl, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₃-C₈)-Cycloalkyl, O-(C₃-C₈)-Cycloalkyl, (C₂-C₆)-Alkinyl, (C₀-C₈)-Alkylen-Aryl, O-(C₀-C₈)-Alkylen-Aryl, S-Aryl, (C₀-C₈)-Alkylen-Heteroaryl, N(R4)(R5), SO₂-CH₃, SO₂-NH₂, SF₅, COOH, COO-(C₁-C₆)-Alkyl, CON(R6)(R7), N(R8)CO(R9), N(R10)SO₂(R11), CO(R12), (CR13R14)ₓ-O(R15), O-CO-N(R16)(R17), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R18)(R19) substituiert sein kann, wobei Aryl oder Heteroaryl wiederum durch
F, Cl, Br, I, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₄)-Haloalkyl (C₂-C₆)-Alkenyl, (C₃-C₈)-Cycloalkyl, O-(C₃-C₈)-Cycloalkyl, (C₂-C₆)-Alkinyl, N(R4a)(R5a), SO₂-CH₃, SF₅, COOH, COO-(C₁-C₆)-Alkyl, CON(R6a)(R7a), N(R8a)CO(R9a), N(R10a)SO₂(R11a), CO(R12a), (CR13aR14a)ₓ-O(R15a), O-CO-N(R16a)(R17a), O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-N(R18a)(R19a) ein oder mehrfach substituiert sein kann;
x, x' 0, 1, 2, 3, 4, 5, 6;
R4, R5, R6, R7, R9, R10, R11, R12, R13, R14, R15, R16, R17, R18, R19, R4a, R5a, R6a, R7a, R9a, R10a, R11a, R12a, R13a, R14a, R15a, R16a, R17a, R18a, R19a
unabhängig voneinander Wasserstoff, (C₁-C₈)-Alkyl;
Y, Z gleich oder verschieden (C₁-C₂)-Alkylen, welches durch F, Cl, CH₃ oder OH einfach substituiert sein kann;
R2 Wasserstoff, (C₁-C₁₂)-Alkyl, Z-Aryl, wobei Aryl oder Heteroaryl gegebenenfalls substituiert sein kann, (C₃-C₁₂)-Cycloalkyl;
R3 (C₁-C₁₂)-Alkyl, Aryl, Heteroaryl, wobei Aryl oder Heteroaryl gegebenenfalls substituiert sein kann, (C₃-C₁₂)-Cycloalkyl; oder
R2 und R3 zusammen mit dem sie tragenden Kohlenstoffatomen bilden ein monocyclisches, gesättigtes oder teilweise ungesättigtes 4- bis 8-gliedriges Ringsystem, deren einzelne Glieder durch ein bis drei Atome oder Atomgruppen aus der Reihe -CHR64-, -CR64R65-, =(C-R66)-,-NR67-, -C(=O)-, -O- ersetzt sein können, mit der Maßgabe, dass zwei Einheiten aus der Reihe -O- nicht benachbart sein dürfen;
R64, R65, R66, R67 gleich oder verschieden Wasserstoff, F, Cl, Br, I, OH, CF₃, NO₂, CN, OCF₃, SF₅, O-(C₁-C₆)-Alkyl, O-(C₁-C₄)-Alkoxy-(C₁-C₄)-Alkyl, S-(C₁-C₆)-Alkyl, (C₁-C₆)-Alkyl, (C₂-C₄)-Haloalkyl, O-(C₂-C₄)-Haloalkyl, (C₂-C₆)-Alkenyl, (C₃-C₈)-Cycloalkyl, O-(C₃- C₈)-Cycloalkyl, (C₃-C₈)-Cycloalkenyl, (C₂-C₆)-Alkinyl, N(R68)(R69), SO₂-CH₃, COOH, COO-(C₁-C₆)-Alkyl, CON(R70)(R71), N(R72)CO(R73), N(R74)SO₂(R75), CO(R76), (CR77R78)_{x""}-O(R79), O-CO-N(R80)(R81), O-CO-(C₁-C₆)- Alkylen-CO-O-(C₁-C₆)-Alkyl, O-CO-(C₁-C₆)-Alkylen-CO-OH, O- CO-(C₁-C₆)-Alkylen-CO-N(R82)(R83);
x"" 0, 1, 2, 3, 4, 5, 6;
R68, R69, R70, R71, R72, R73, R74, R75, R76, R77, R78, R79, R80, R81, R82, R83
gleich oder verschieden Wasserstoff, (C₁-C₆)-Alkyl;
die tautomeren Formen der Verbindung sowie deren physiologisch verträgliche Salze.

2. Verbindung der Formel I gemäß Anspruch 1, **dadurch gekennzeichnet, dass**
R1 Y-Phenyl, Y-Heteroaryl, wobei Heteroaryl 1 Heteroatom aus der Reihe N, O, S enthält und wobei Phenyl oder Heteroaryl durch F, Cl, Br, OH, CF₃, NO₂, CN, OCF₃, O-(C₁-C₆)-Alkyl, S-(C₁-C₆)-Alkyl, (C₁-C₆)-Alkyl, (C₂-C₄)-Haloalkyl, (C₃-C₈)-Cycloalkyl, (C₀-C₆)-Alkylen-Phenyl, O-(C₀-C₆)-Alkylen-Phenyl, S-Phenyl, (C₀-C₈)-Alkylen-Heteroaryl, N(R4)(R5), COOH, COO-(C₁-C₆)-Alkyl, CON(R6)(R7), CO(R12), ein- oder mehrfach substituiert sein kann, wobei Phenyl oder Heteroaryl wiederum durch
F, Cl, OH, CF₃, NO₂, CN, OCF₃, O-(C₁-C₆)-Alkyl, S-(C₁-C₆)- Alkyl, (C₁-C₆)-Alkyl, (C₂-C₄)-Haloalkyl, (C₃-C₈)-Cycloalkyl, N(R4a)(R5a), COOH, COO-(C₁-C₆)-Alkyl, CON(R6a)(R7a) CO(R12a) ein oder mehrfach substituiert sein kann;
x, x' 0, 1, 2, 3, 4, 5, 6;
R4, R5, R6, R7, R12, R4a, R5a, R6a, R7a, , R12a, gleich oder verschieden Wasserstoff, (C₁-C₈)-Alkyl;
oder ein Rest der Formel Ib 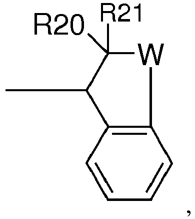 mit
W - C(R26)(R27)-, -C(R26)(R27)-C(R28)(R29)-, -C(R26)(R27)-O-;
R20, R21, R26, R27, R28, R29 gleich oder verschieden Wasserstoff, F, Cl, OH, CF₃, NO₂, CN, OCF₃, O-(C₁-C₆)-Alkyl, (C₁-C₆)-Alkyl, CO-(C₁-C₆)-Alkyl;
oder
R20, R21, R26 und R27 zusammen mit dem sie tragenden Kohlenstoffatomen bilden einen annelierten Benzolrest, welcher durch F, Cl, CN, NO₂, CF₃, OCF₃, (C₁-C₆)-Alkyl, O-(C₁-C₆)-Alkyl, CO-(C₁-C₆)-Alkyl ein oder mehrfach substituiert sein kann;
Y, Z gleich oder verschieden -CH₂- oder -CH₂-CH₂-, welches durch CH₃ oder OH einfach substituiert sein kann;
R2 Wasserstoff, (C₁-C₁₂)-Alkyl, Z-Phenyl, wobei Phenyl gegebenenfalls substituiert sein kann, (C₃-C₁₂)-Cycloalkyl;
R3 (C₁-C₁₂)-Alkyl, Phenyl, Heteroaryl, welches 1 Heteroatom aus der Reihe N, O, S enthält, wobei Phenyl oder Heteroaryl gegebenenfalls substituiert sein kann, (C₃-C₁₂)-Cycloalkyl; oder
R2 und R3 zusammen mit dem sie tragenden Kohlenstoffatomen bilden ein monocyclisches, gesättigtes 5- bis 7-gliedriges Ringsystem, dessen einzelne Glieder durch ein bis drei Atomgruppen aus der Reihe -CHR64-, -CR64R65-, =(C-R66)- ersetzt sein können;
R64, R65, R66 gleich oder verschieden F, Cl, OH, CF₃, O-(C₁-C₆)-Alkyl, O-(C₁- C₄)-Alkoxy-(C₁-C₄)-Alkyl, (C₁-C₆)-Alkyl, (C₂-C₄)-Haloalkyl, (C₃- C₈)-Cycloalkyl, N(R68)(R69), SO₂-CH₃, COOH, COO-(C₁-C₆)- Alkyl, CON(R70)(R71), N(R72)CO(R73), CO(R76), O-CO- N(R80)(R81), O-CO-(C₁-C₆)-Alkylen-CO-O-(C₁-C₆)-Alkyl, O- CO-(C₁-C₆)-Alkylen-CO-OH, O-CO-(C₁-C₆)-Alkylen-CO- N(R82)(R83);
R68, R69, R70, R71, R72, R73, R76, R77, R78, R79, R80, R81, R82, R83
gleich oder verschieden Wasserstoff, (C₁-C₆)-Alkyl;
bedeuten,
die tautomeren Formen der Verbindung sowie deren physiologisch verträgliche Salze.

3. Verbindung der Formel I gemäß Anspruch 1 oder 2, **dadurch gekennzeichnet, dass**
R1 Y-Phenyl, Y-Pyridyl, Y-Thienyl, Y-Furyl, Y-Benzothienyl, Y-Benzofuryl, wobei Phenyl oder der heteroaromatische Rest durch F, Cl, Br, CF₃, CN, OCF₃, O-(C₁-C₆)-Alkyl, (C₁-C₆)-Alkyl, (C₃-C₆)-Cycloalkyl, N(R4)(R5), COOH, COO-(C₁-C₆)-Alkyl, CON(R6)(R7), CO(R12) ein-, zwei- oder dreifach substituiert und durch (C₀-C₁)-Alkylen-Phenyl, O-(C₀-C₁)-Phenyl, Pyrazolyl, Pyridyl, Thienyl, Furyl, Benzothienyl, Benzofuryl einfach substituiert sein kann, wobei ein heteroaromatische Rest oder Phenyl wiederum durch F, Cl, Br, CF₃, CN, OCF₃, O-(C₁-C₆)-Alkyl, (C₁-C₆)-Alkyl, (C₃-C₆)-Cycloalkyl, N(R4a)(R5a), COOH, COO-(C₁-C₆)-Alkyl, CON(R6a)(R7a), CO(R12a) ein-, zwei oder dreifach substituiert sein kann;
R4, R5, R6, R7, R12, R4a, R5a, R6a, R7a, R12a unabhängig voneinander H, (C₁-C₆)-Alkyl;
Y -CH₂- oder -CH₂-CH₂-, welches durch CH₃ einfach substituiert sein kann;
R2 Wasserstoff, (C₁-C₆)-Alkyl, -CH₂-Phenyl, wobei Phenyl gegebenenfalls substituiert sein kann, (C₃-C₈)-Cycloalkyl;
R3 (C₁-C₈)-Alkyl, Phenyl, Pyridyl, Thienyl, wobei Phenyl, Pyridyl oder Thienyl gegebenenfalls substituiert sein kann, (C₃-C₈)-Cycloalkyl; oder
R2 und R3 zusammen mit dem sie tragenden Kohlenstoffatomen bilden ein monocyclisches, gesättigtes 6- bis 7-gliedriges Ringsystem, deren einzelne Glieder durch ein bis drei Atome oder Atomgruppen aus der Reihe -CHR64-, -CR64R65-, ersetzt sein können;
R64, R65 gleich oder verschieden F, Cl, CF₃, OCF₃, (C₁-C₆)-Alkyl, O-(C₁-C₆)-Alkyl, N(R68)(R69), COOH, COO-(C₁-C₆)-Alkyl, CO-N(R70)(R71), CO(R76);
R68, R69, R70, R71, R76 gleich oder verschieden Wasserstoff, (C₁-C₆)-Alkyl;
bedeuten,
die tautomeren Formen der Verbindung sowie deren physiologisch verträgliche Salze.

4. Verbindung der Formel I gemäß Ansprüchen 1 bis 3, **dadurch gekennzeichnet, dass**
R2 Isopropyl und
R3 Methyl bedeutet.

5. Verbindung der Formel I gemäß Ansprüchen 1 bis 3, **dadurch gekennzeichnet, dass**
R2 Wasserstoff und
R3 Phenyl, welches durch Cl einfach substituiert sein kann
bedeutet.

6. Verbindung der Formel I gemäß Ansprüchen 1 bis 3, **dadurch gekennzeichnet, dass**
R2 und R3 zusammen -CH₂-CH₂-CH₂-CH₂- oder -CH₂-CH₂-CH₂-CH₂-CH₂-.
bedeutet.

7. Verbindung der Formel I gemäß Ansprüchen 1 bis 3, **dadurch gekennzeichnet, dass**
R1 Y-Phenyl, Y-Thienyl, Y-Benzothienyl, wobei Phenyl oder der heteroaromatische Rest durch F, Cl, Br, CF₃, O-CH₃, -CH₃, -CH₂CH₃,-CH₂-CH₂-CH₂-CH₃, ein-, zwei- oder dreifach substituiert und durch Phenyl, Pyrazolyl oder Thienyl einfach substituiert sein kann,
wobei der heteroaromatische Rest oder Phenyl wiederum durch F, Cl, Br, CF₃, O-(C₁-C₆)-Alkyl, (C₁-C₆)-Alkyl ein-, zwei- oder dreifach substituiert sein kann;
Y -CH₂-, welches durch CH₃ einfach substituiert sein kann;
R2 Wasserstoff, Methyl, Isopropyl, Cyclopropyl, -CH₂-Phenyl, wobei Phenyl durch Cl in 4-Position substituiert sein kann;
R3 Methyl, Phenyl, Pyridyl, Cyclopropyl, wobei Phenyl durch Cl substituiert sein kann; oder
R2 und R3 zusammen -CH₂-CH₂-CH₂-CH₂- oder -CH₂-CH₂-CH₂-CH₂-CH₂;
bedeuten,
die tautomeren Formen der Verbindung sowie deren physiologisch verträgliche Salze.

8. Arzneimittel enthaltend eine oder mehrere Verbindungen der Formel I gemäß den Ansprüchen 1 bis 7.

9. Verwendung der Verbindungen der Formel I gemäß den Ansprüchen 1 bis 7 zur Herstellung eines Medikaments zur Behandlung und/oder Prävention von Störungen des Fettsäurestoffwechsels und Glucoseverwertungsstörungen.

10. Verwendung der Verbindungen der Formel I gemäß den Ansprüchen 1 bis 7 zur Herstellung eines Medikaments zur Behandlung und/oder Prävention von Störungen, bei denen Insulin Resistenz eine Rolle spielt.

11. Verwendung der Verbindungen der Formel I gemäß den Ansprüchen 1 bis 7 zur Herstellung eines Medikaments zur Behandlung und/oder Prävention von Diabetes mellitus und der damit verbundenen Folgeerkrankungen.

12. Verwendung der Verbindungen der Formel I gemäß den Ansprüchen 1 bis 7 zur Herstellung eines Medikaments zur Behandlung und/oder Prävention von Dyslipidämien und deren Folgen.

13. Verwendung der Verbindungen der Formel I gemäß den Ansprüchen 1 bis 7 zur Herstellung eines Medikaments zur Behandlung und/oder Prävention von Zuständen, die mit dem Metabolischen Syndrom assoziert sind.

14. Verwendung der Verbindungen der Formel I gemäß den Ansprüchen 1 bis 7 zur Herstellung eines Medikaments zur Behandlung und/oder Prävention von Zuständen, die mit erniedrigtem HDL-Spiegel verbunden sind.

15. Verwendung der Verbindungen der Formel I gemäß den Ansprüchen 1 bis 7 zur Herstellung eines Medikaments zur Behandlung und/oder Prävention von atherosklerotischen Erkrankungen.

16. Verwendung der Verbindungen der Formel I gemäß den Ansprüchen 1 bis 7 in Kombination mit mindestens einem weiteren Wirkstoff zur Herstellung eines Medikaments zur Behandlung und/oder Prävention von Störungen, bei denen Insulin Resistenz eine Rolle spielt.

17. Verfahren zur Herstellung eines Arzneimittels enthaltend eine oder mehrere der Verbindungen der Formel I gemäß den Ansprüchen 1 bis 7, **dadurch gekennzeichnet, dass** diese mit einem pharmazeutisch geeigneten Träger vermischt wird und diese Mischung in eine für die Verabreichung geeignete Form gebracht wird.

18. Verfahren zur Herstellung von Verbindungen der allgemeinen Formel I gemäß den Ansprüchen 1 bis 7, **dadurch gekennzeichnet, dass** 3-Oxoisoxazolderivate der Formel II
a) mit Carbamoylchloriden der Formel III acyliert werden;
oder
b) in zwei Stufen zunächst mit Phosgen oder Äquivalenten wie Chlorcarbonsäuretrichlormethylester, Carbonsäureditrichlormethylester oder Chlorameisensäure-4-nitrophenylester und einem zweiten Schritt mit Aminen der Formel IV umgesetzt werden, oder
c) mit Isocyanaten der Formel V: O=C=N-R1 umgesetzt werden,
worin die Substituenten die oben genannten Bedeutungen haben.

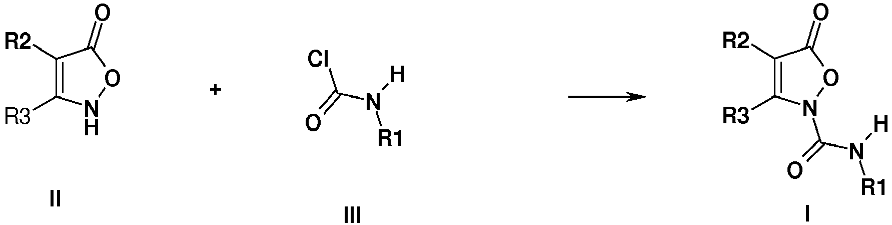

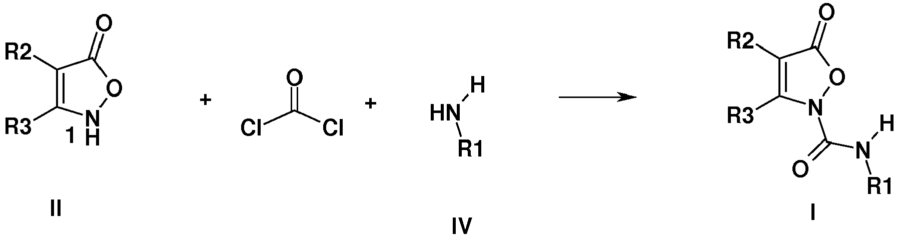

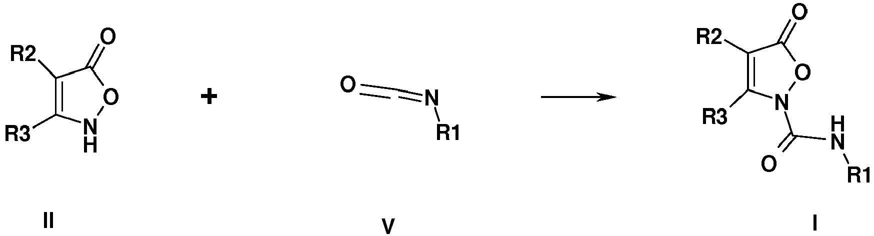

## Claims

1. A compound of the formula I 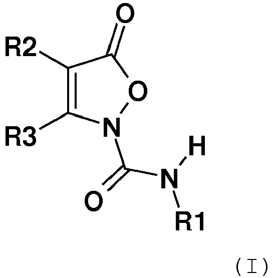 in which the meanings are:
R1 Y-aryl, Y-heteroaryl,
where aryl or heteroaryl may be substituted one or more times by F, Cl, Br, I, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₄)-haloalkyl, O-(C₂-C₄)-haloalkyl, (C₂-C₆)-alkenyl, (C₃-C₈)-cycloalkyl, O-(C₃-C₈)-cycloalkyl, (C₂-C₆)-alkynyl, (C₀-C₈)-alkylene-aryl, O-(C₀-C₈)-alkylene-aryl, S-aryl, (C₀-C₈)-alkylene-heteroaryl, N(R4)(R5), SO₂-CH₃, SO₂-NH₂, SF₅, COOH, COO-(C₁-C₆)-alkyl, CON(R6)(R7), N(R8)CO(R9), N(R10)SO₂(R11), CO(R12), (CR13R14)ₓ-O(R15), O-CO-N(R16) (R17), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R18)(R19), where aryl or heteroaryl may in turn be substituted one or more times by
F, Cl, Br, I, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₄)-haloalkyl, (C₂-C₆)-alkenyl, (C₃-C₈)-cycloalkyl, O-(C₃-C₈)-cycloalkyl, (C₂-C₆)-alkynyl, N(R4a)(R5a), SO₂-CH₃, SF₅, COOH, COO-(C₁-C₆)-alkyl, CON(R6a)(R7a), N(R8a)CO(R9a), N(R10a)SO₂(R11a), CO(R12a), (CR13aR14a)_{x'}-O(R15a), O-CO-N(R16a)(R17a), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R18a)(R19a) ;
x, x' 0, 1, 2, 3, 4, 5, 6;
R4, R5, R6, R7, R9, R10, R11, R12, R13, R14, R15, R16, R17, R18, R19,
R4a, R5a, R6a, R7a, R9a, R10a, R11a, R12a, R13a, R14a, R15a, R16a, R17a, R18a, R19a
independently of one another hydrogen, (C₁-C₈)-alkyl;
Y, Z identically or differently (C₁-C₂)-alkylene, which may be substituted once by F, Cl, CH₃ or OH;
R2 hydrogen, (C₁-C₁₂)-alkyl, Z-aryl, where aryl or heteroaryl may optionally be substituted, (C₃-C₁₂)-cycloalkyl;
R3 (C₁-C₁₂)-alkyl, aryl, heteroaryl, where aryl or heteroaryl may optionally be substituted, (C₃-C₁₂)-cycloalkyl; or
R2 and R3 together with the carbon atoms carrying them form a monocyclic, saturated or partly unsaturated 4-to 8-membered ring system whose individual members may be replaced by one to three atoms or atomic groups from the series -CHR64-, -CR64R65-, =(C-R66)-, -NR67-, -C(=O)-, -0-, with the proviso that two units from the series -0- may not be adjacent;
R64, R65, R66, R67 identically or differently hydrogen, F, Cl, Br, I, OH, CF₃, NO₂, CN, OCF₃, SF₅, 0-(C₁-C₆)-alkyl, O-(C₁-C₄)-alkoxy-(C₁-C₄)-alkyl, S-(C₁-C₆)-alkyl, (C₁-C₆)-alkyl, (C₂-C₄)-haloalkyl, O-(C₂-C₄)-haloalkyl, (C₂-C₆)-alkenyl, (C₃-C₈)-cycloalkyl, O-(C₃-C₈)-cycloalkyl, (C₃-C₈)-cycloalkenyl, (C₂-C₆)-alkynyl, N(R68)(R69), SO₂-CH₃, COOH, COO-(C₁-C₆)-alkyl, CON(R70)(R71), N(R72)CO(R73), N(R74)SO₂(R75), CO(R76), (CR77R78)ₓ""-O(R79), O-CO-N(R80) (R81), O-CO-(C₁-C₆)-alkylene-CO-O-(C₁-C₆)-alkyl, O-CO-(C₁-C₆)-alkylene-CO-OH, O-CO-(C₁-C₆)-alkylene-CO-N(R82)(R83);
x"" 0, 1, 2, 3, 4, 5, 6;
R68, R69, R70, R71, R72, R73, R74, R75, R76, R77, R78, R79, R80, R81, R82, R83
identically or differently hydrogen, (C₁-C₆)-alkyl;
the tautomeric forms of the compound and the physiologically tolerated salts thereof.

2. A compound of the formula I as claimed in claim 1, wherein
R1 is Y-phenyl, Y-heteroaryl, where heteroaryl comprises 1 heteroatom from the series N, 0, S, and where phenyl or heteroaryl may be substituted one or more times by F, Cl, Br, OH, CF₃, NO₂, CN, OCF₃, O-(C₁-C₆)-alkyl, S-(C₁-C₆)-alkyl, (C₁-C₆)-alkyl, (C₂-C₄)-haloalkyl, (C₃-C₈)-cycloalkyl, (C₀-C₆)-alkylene-phenyl, O-(C₀-C₆)-alkylene-phenyl, S-phenyl, (C₀-C₈)-alkylene-heteroaryl, N(R4)(R5), COOH, COO-(C₁-C₆)-alkyl, CON(R6)(R7), CO(R12), where phenyl or heteroaryl may in turn be substituted one or more times by
F, Cl, OH, CF₃, NO₂, CN, OCF₃, O-(C₁-C₆)-alkyl, S-(C₁-C₆)-alkyl, (C₁-C₆)-alkyl, (C₂-C₄)-haloalkyl, (C₃-C₈)-cycloalkyl, N(R4a)(R5a), COOH, COO-(C₁-C₆)-alkyl, CON(R6a)(R7a) CO(R12a);
x, x' are 0, 1, 2, 3, 4, 5, 6;
R4, R5, R6, R7, R12, R4a, R5a, R6a, R7a, R12a are
identically or differently hydrogen, (C₁-C₈)-alkyl;
or a radical of the formula Ib 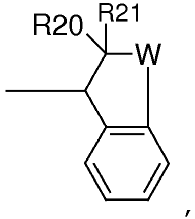 with
W is -C(R26)(R27)-, -C(R26)(R27)-C(R28)(R29)-, -C(R26)(R27)-O-;
R20, R21, R26, R27, R28, R29 are
identically or differently hydrogen, F, Cl, OH, CF₃, NO₂, CN, OCF₃, O-(C₁-C₆)-alkyl, (C₁-C₆)-alkyl, CO-(C₁-C₆)-alkyl;
or
R20, R21, R26 and R27 together with the carbon atoms carrying them form a fused benzene residue which may be substituted one or more times by F, Cl, CN, NO₂, CF₃, OCF₃, (C₁-C₆)-alkyl, O-(C₁-C₆)-alkyl, CO-(C₁-C₆)-alkyl;
Y, Z are identically or differently -CH₂- or -CH₂-CH₂-, which may be substituted once by CH₃ or OH;
R2 is hydrogen, (C₁-C₁₂)-alkyl, Z-phenyl, where phenyl may optionally be substituted, (C₃-C₁₂)-cycloalkyl;
R3 is (C₁-C₁₂)-alkyl, phenyl, heteroaryl, which comprises 1 heteroatom from the series N, 0, S, where phenyl or heteroaryl may optionally be substituted, (C₃-C₁₂)-cycloalkyl; or
R2 and R3 together with the carbon atoms carrying them form a monocyclic, saturated 5- to 7-membered ring system whose individual members may be replaced by one to three atomic groups from the series -CHR64-, - CR64R65-, =(C-R66)-;
R64, R65, R66 are identically or differently F, Cl, OH, CF₃, O-(C₁-C₆)-alkyl, O-(C₁-C₄)-alkoxy-(C₁-C₄)-alkyl, (C₁-C₆)-alkyl, (C₂-C₄)-haloalkyl, (C₃-C₈)-cycloalkyl, N(R68)(R69), SO₂-CH₃, COOH, COO-(C₁-C₆)-alkyl, CON(R70)(R71), N(R72)CO(R73), CO(R76), O-CO-N(R80)(R81), O-CO-(C₁-C₆)-alkylene-CO-O-(C₁-C₆)-alkyl, 0-CO-(C₁-C₆)-alkylene-CO-OH, O-CO-(C₁-C₆)-alkylene-CO-N(R82)(R83);
R68, R69, R70, R71, R72, R73, R76, R77, R78, R79, R80, R81, R82, R83 are
identically or differently hydrogen, (C₁-C₆)-alkyl;
the tautomeric forms of the compound, and the physiologically tolerated salts thereof.

3. A compound of the formula I as claimed in claim 1 or 2, wherein
R1 is Y-phenyl, Y-pyridyl, Y-thienyl, Y-furyl, Y-benzothienyl, Y-benzofuryl, where phenyl or the heteroaromatic radical may be substituted once, twice or three times by F, Cl, Br, CF₃, CN, OCF₃, O-(C₁-C₆)-alkyl, (C₁-C₆)-alkyl, (C₃-C₆)-cycloalkyl, N(R4)(R5), COOH, COO-(C₁-C₆)-alkyl, CON(R6)(R7), CO(R12), and may be substituted once by (C₀-C₁)-alkylene-phenyl, O-(C₀-C₁)-phenyl, pyrazolyl, pyridyl, thienyl, furyl, benzothienyl, benzofuryl, where a heteroaromatic radical or phenyl may in turn be substituted once, twice or three times by F, Cl, Br, CF₃, CN, OCF₃, O-(C₁-C₆)-alkyl, (C₁-C₆)-alkyl, (C₃-C₆)-cycloalkyl, N(R4a)(R5a), COOH, COO-(C₁-C₆)-alkyl, CON(R6a)(R7a), CO(R12a);
R4, R5, R6, R7, R12, R4a, R5a, R6a, R7a, R12a
are independently of one another H, (C₁-C₈)-alkyl;
Y is -CH₂- or -CH₂-CH₂-, which may be substituted once by CH₃;
R2 is hydrogen, (C₁-C₈)-alkyl, -CH₂-phenyl, where phenyl may optionally be substituted, (C₃-C₈)-cycloalkyl;
R3 is (C₁-C₈)-alkyl, phenyl, pyridyl, thienyl, where phenyl, pyridyl or thienyl may optionally be substituted, (C₃-C₈)-cycloalkyl; or
R2 and R3 together with the carbon atoms carrying them form a monocyclic, saturated 6- to 7-membered ring system whose individual members may be replaced by one to three atoms or atomic groups from the series -CHR64-, -CR64R65-;
R64, R65 are identically or differently F, Cl, CF₃, OCF₃, (C₁-C₆)-alkyl, O- (C₁-C₆)-alkyl, N(R68)(R69), COOH, COO-(C₁-C₆)-alkyl, CO-N(R70)(R71), CO(R76);
R68, R69, R70, R71, R76 are identically or differently hydrogen, (C₁-C₆)-alkyl;
the tautomeric forms of the compound, and the physiologically tolerated salts thereof.

4. A compound of the formula I as claimed in claims 1 to 3, wherein
R2 is isopropyl and
R3 is methyl.

5. A compound of the formula I as claimed in claims 1 to 3, wherein
R2 is hydrogen and
R3 is phenyl, which may be substituted once by Cl.

6. A compound of the formula I as claimed in claims 1 to 3, wherein
R2 and R3 together are -CH₂-CH₂-CH₂-CH₂- or -CH₂-CH₂-CH₂-CH₂-CH₂-.

7. A compound of the formula I as claimed in claims 1 to 3, wherein
R1 is Y-phenyl, Y-thienyl, Y-benzothienyl, where phenyl or the heteroaromatic radical may be substituted once, twice or three times by F, Cl, Br, CF₃, O-CH₃, - CH₃, -CH₂CH₃, -CH₂-CH₂-CH₂-CH₃, and be substituted once by phenyl, pyrazolyl or thienyl,
where the heteroaromatic radical or phenyl may in turn be substituted once, twice or three times by F, Cl, Br, CF₃, O-(C₁-C₆)-alkyl, (C₁-C₆)-alkyl;
Y is -CH₂-, which may be substituted once by CH₃;
R2 is hydrogen, methyl, isopropyl, cyclopropyl, -CH₂-phenyl, where phenyl may be substituted by Cl in position 4;
R3 is methyl, phenyl, pyridyl, cyclopropyl, where phenyl may be substituted by Cl; or
R2 and R3 are together -CH₂-CH₂-CH₂-CH₂- or -CH₂-CH₂-CH₂-CH₂-CH₂;
the tautomeric forms of the compound, and the physiologically tolerated salts thereof.

8. A medicament comprising one or more compounds of the formula I as claimed in claims 1 to 7.

9. The use of the compounds of the formula I as claimed in claims 1 to 7 for the manufacture of a medicament for the treatment and/or prevention of disorders of fatty acid metabolism and glucose utilization disorders.

10. The use of the compounds of the formula I as claimed in claims 1 to 7 for the manufacture of a medicament for the treatment and/or prevention of disorders in which insulin resistance is involved.

11. The use of the compounds of the formula I as claimed in claims 1 to 7 for the manufacture of a medicament for the treatment and/or prevention of diabetes mellitus and the sequelae associated therewith.

12. The use of the compounds of the formula I as claimed in claims 1 to 7 for the manufacture of a medicament for the treatment and/or prevention of dyslipidemias and the sequelae thereof.

13. The use of the compounds of the formula I as claimed in claims 1 to 7 for the manufacture of a medicament for the treatment and/or prevention of conditions associated with the metabolic syndrome.

14. The use of the compounds of the formula I as claimed in claims 1 to 7 for the manufacture of a medicament for the treatment and/or prevention of conditions associated with reduced HDL level.

15. The use of the compounds of the formula I as claimed in claims 1 to 7 for the manufacture of a medicament for the treatment and/or prevention of atherosclerotic disorders.

16. The use of the compounds of the formula I as claimed in claims 1 to 7 in combination with at least one further active ingredient for the manufacture of a medicament for the treatment and/or prevention of disorders in which insulin resistance is involved.

17. A process for the manufacture of a medicament comprising one or more of the compounds of the formula I as claimed in claims 1 to 7, which comprises mixing the latter with a pharmaceutically suitable carrier, and converting this mixture into a form suitable for administration.

18. A process for preparing compounds of the formula I as claimed in claims 1 to 7, which comprises 3-oxoisoxazole derivatives of the formula II
a) being acylated with carbamoyl chlorides of the formula III;
or
b) in two stages being reacted first with phosgene or equivalents such as trichloromethyl chlorocarbonate, ditrichloromethyl carbonate or 4-nitrophenyl chloroformate and in a second step with amines of the formula IV, or
c) being reacted with isocyanates of the formula V: O=C=N-R1,
in which the substituents have the abovementioned meanings.

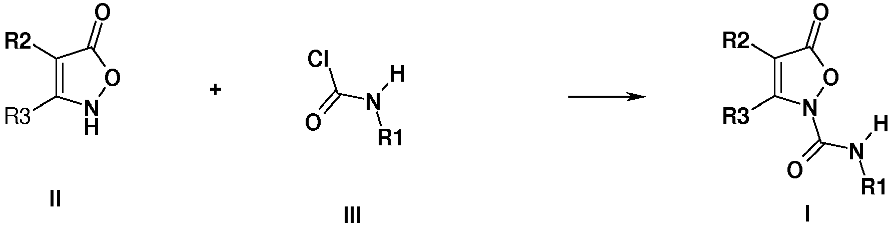

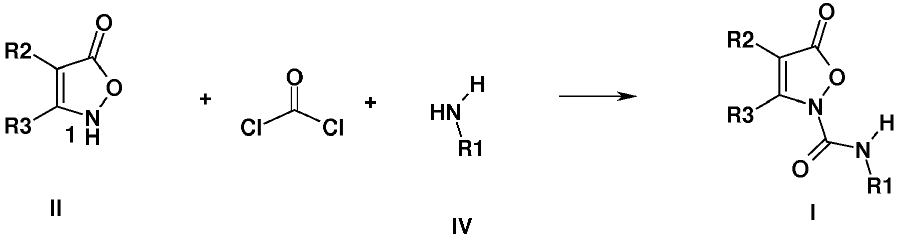

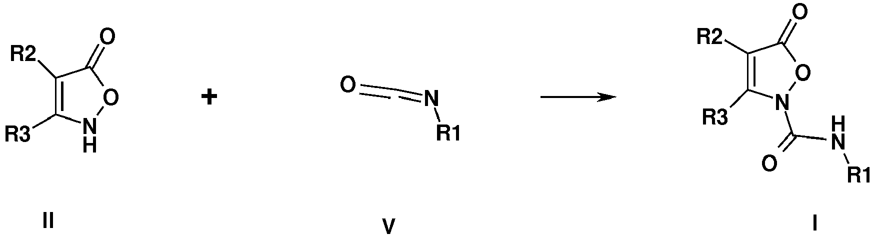

## Revendications

1. Composé de formule générale I 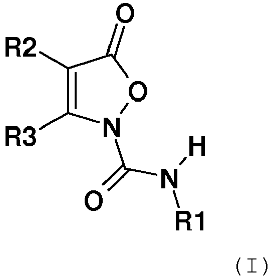 dans lequel:
R1 est un Y-aryle, Y-hétéroaryle, où l'aryle ou l'hétéroaryle peuvent être substitués une ou plusieurs fois par F, Cl, Br, I, OH, CF₃, NO₂, CN, OCF₃, O-(alkyle en C₁-C₆), O-(alcoxy en C₁-C₄)-(alkyle en C₁-C₄), S-(alkyle en C₁-C₆), un alkyle en C₁-C₆, un halogénoalkyle en C₂-C₄, O-(halogénoalkyle en C₂-C₄), un alcényle en C₂-C₆, un cycloalkyle en C₃-C₈, O-(cycloalkyle en C₃-C₈), un alcynyle en C₂-C₆, un (alkylène en C₀-C₈)-aryle, O-(alkylène en C₀-C₈)-aryle, S-aryle, un (alkylène en C₀-C₈)-hétéroaryle, N(R4)(R5), SO₂-CH₃, SO₂-NH₂, SF₅, COOH, COO-(alkyle en C₁-C₆), CON(R6)(R7), N(R8)CO(R9), N(R10)SO₂(R11), CO(R12), (CR13R14)ₓ-O(R15), O-CO-N(R16)(R17), O-CO-(alkylène en C₁-C₆)-CO-O-(alkyle en C₂-C₆), O-CO-(alkylène en C₁-C₆)-CO-OH, O-CO-(alkylène en C₁-C₆)-CO-N(R18)(R19), où l'aryle ou l'hétéroaryle peuvent eux-mêmes être substitués une ou plusieurs fois par
F, Cl, Br, I, OH, CF₃, NO₂, CN, OCF₃, O- (alkyle en C₁-C₆), O-(alcoxy en C₁-C₄)-(alkyle en C₁-C₄), S-(alkyle en C₁-C₆), un alkyle en C₁-C₆, un halogénoalkyle en C₂-C₄, un alcényle en C₂-C₆, un cycloalkyle en C₃-C₈, O- (cycloalkyle en C₃-C₈), un alcynyle en C₂-C₆, N(R4a)(R5a), SO₂-CH₃, SF₅, COOH, COO-(alkyle en C₁-C₆), CON(R6a)(R7a), N(R8a)CO(R9a), N(R10a)SO₂(R11a), CO(R12a), (CR13aR14a)_{x'}- O(R15a), O-CO-N(R16a)(R17a), O-CO-(alkylène en C₁-C₆)-CO-O-(alkyle en C₁-C₆), O-CO- (alkylène en C₁-C₆)-CO-OH, O-CO-(alkylène en C₁-C₆)-CO-N(R18a)(R19a) ;
x, x' 0, 1, 2, 3, 4, 5, 6 ;
R4, R5, R6, R7, R9, R10, R11, R12, R13, R14, R15, R16, R17, R18, R19,
R4a, R5a, R6a, R7a, R9a, R10a, R11a, R12a, R13a, R14a, R15a, R16a, R17a, R18a, R19a
sont, indépendamment les un des autres, un hydrogène, un alkyle en C₁-C₈ ;
Y, Z sont, identiques ou différents, un alkylène en C₁-C₂, qui peut être substitué une fois par F, Cl, CH₃ ou OH ;
R2 un hydrogène, un alkyle en C₁-C₁₂, Z-aryle, où l'aryle ou l'hétéroaryle peutvent facultativement être substitués, un cycloalkyle en C₃-C₁₂ ;
R3 un alkyle en C₁-C₁₂, un aryle, un hétéroaryle, où l'aryle ou l'hétéroaryle peuvent facultativement être substitués, un cycloalkyle en C₃-C₁₂ ; ou
R2 et R3 conjointement avec les atomes de carbone portant ceux-ci forment un système cyclique monocyclique, saturé ou partiellement insaturé, à 4 à 8 chaînons, dont les chaînons individuels peuvent être remplacés par un à trois atomes ou groupes atomiques de la série -CHR64-, -CR64R65-, =(C-R66)-, -NR67-, -C(=O)-, -O-, à condition que deux motifs de la série -O- ne soient pas adjacents ;
R64, R65, R66, R67 sont, identiques ou différents, un hydrogène, F, Cl, Br, I, OH, CF₃, NO₂, CN, OCF₃, SF₅, O-(alkyle en C₁-C₆), O-(alcoxy en C₁-C₄)-(alkyle en C₁-C₄), S-(alkyle en C₁-C₆), un alkyle en C₁-C₆, un halogénoalkyle en C₂-C₄, O-(halogénoalkyle en C₂-C₄), un alcényle en C₂-C₆, un cycloalkyle en C₃-C₈, O-(cycloalkyle en C₃-C₈), un cycloalcényle en C₃-C₈, un alcynyle en C₂-C₆, N (R68) (R69) , SO₂-CH₃, COOH, COO-(alkyle en C₁-C₆), CON(R70)(R71), N(R72)CO(R73), N(R74)SO₂(R75), CO(R76), (CR77R78)_{x''''}-O(R79), O-CO-N(R80)(R81), O-CO-(alkylène en C₁-C₆)-CO-O-(alkyle en C₁-C₆), O-CO-(alkylène en C₁-C₆)-CO-OH, O-CO-(alkylène en C₁-C₆)-CO-N(R82)(R83) ;
x"" 0, 1, 2, 3, 4, 5, 6 ;
R68, R69, R70, R71, R72, R73, R74, R75, R76, R77, R78, R79, R80, R81, R82, R83
sont, identiques ou différents, un hydrogène, un alkyle en C₁-C₆ ;
les formes tautomères des composés et leurs sels physiologiquement acceptables.

2. Composé de formule I selon la revendication 1, dans lequel
R1 est un Y-phényle, Y-hétéroaryle, où l'hétéroaryle comprend 1 hétéroatome de la série N, 0, S, et où le phényle ou l'hétéroaryle peutvent être substitués une ou plusieurs fois par F, Cl, Br, OH, CF₃, NO₂, CN, OCF₃, O-(alkyle en C₁-C₆), S-(alkyle en C₁-C₆), un alkyle en C₁-C₆, un halogénoalkyle en C₂-C₄, un cycloalkyle en C₃-C₈, un (alkylène en C₀-C₆) -phényle, O-(alkylène en C₀-C₆) -phényle, S-phényle, un (alkylène en C₀-C₈) -hétéroaryle, N(R4)(R5), COOH, COO-(alkyle en C₁-C₆), CON(R6)(R7), CO(R12), où le phényle ou l'hétéroaryle peuvent eux-mêmes être substitués une ou plusieurs fois par
F, Cl, OH, CF₃, NO₂, CN, OCF₃, O-(alkyle en C₁-C₆) , S-(alkyle en C₁-C₆), un alkyle en C₁-C₆, un halogénoalkyle en C₂-C₄, un cycloalkyle en C₃-C₈, N(R4a)(R5a), COOH, COO- (alkyle en C₁-C₆), CON(R6a)(R7a) CO(R12a) ;
x,x' 0,1,2,3,4,5,6;
R4, R5, R6, R7, R12, R4a, R5a, R6a, R7a, R12a, sont, identiques ou différents, un hydrogène, un alkyle en C₁-C₈ ;
ou un radical de formule Ib 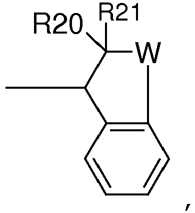 avec
W -C (R26) (R27) -, -C (R26) (R27) -C (R28) (R29) -, -C(R26)(R27)-O- ;
R20, R21, R26, R27, R28, R29
sont, identiques ou différents, un hydrogène, F, Cl, OH, CF₃, NO₂, CN, OCF₃, O-(alkyle en C₁-C₆), un alkyle en C₁-C₆, CO- (alkyle en C₁-C₆) ;
ou
R20, R21, R26 et R27 conjointement avec les atomes de carbone portant ceux-ci forment un résidu benzène condensé qui peut être substitué une ou plusieurs fois par F, Cl, CN, NO₂, CF₃, OCF₃, un alkyle en C₁-C₆, O-(alkyle en C₁-C₆) , CO- (alkyle en C₁-C₆) ;
Y, Z sont, identiques ou différents, -CH₂- ou -CH₂-CH₂-, qui peut être substitué une fois par CH₃ ou OH ;
R2 un hydrogène, un alkyle en C₁-C₁₂, Z-phényle, où le phényle peut facultativement être substitué, un cycloalkyle en C₃-C₁₂ ;
R3 un alkyle en C₁-C₁₂, un phényle, un hétéroaryle, qui comprend 1 hétéroatome de la série N, 0, S, où le phényle ou l'hétéroaryle peuvent facultativement être substitués, un cycloalkyle en C₃-C₁₂ ; ou
R2 et R3 conjointement avec les atomes de carbone portant ceux-ci forment un système cyclique monocyclique, saturé, à 5 à 7 chaînons dont les chaînons individuels peuvent être remplacés par un à trois groupes atomiques de la série -CHR64-, -CR64R65-, =(C-R66)- ;
R64, R65, R66 sont, identiques ou différents, F, Cl, OH, CF₃, O-(alkyle en C₁-C₆), O-(alcoxy en C₁-C₄)-(alkyle en C₁-C₄), un alkyle en C₁-C₆, un halogénoalkyle en C₂-C₄, un cycloalkyle en C₃-C₈, N(R68)(R69), SO₂-CH₃, COOH, COO-(alkyle en C₁-C₆), CON(R70)(R71), N(R72)CO(R73), CO(R76), O-CO-N(R80) (R81), O-CO-(alkylène en C₁-C₆)-CO-O-(alkyle en C₁-C₆), O-CO-(alkylène en C₁-C₆)-CO-OH, O-CO-(alkylène en C₁-C₆)-CO-N(R82)(R83) ;
R68, R69, R70, R71, R72, R73, R76, R77, R78, R79, R80, R81, R82, R83
sont, identiques ou différents, un hydrogène, un alkyle en C₁-C₆ ;
les formes tautomères des composés et leurs sels physiologiquement acceptables.

3. Composé de formule I selon la revendication 1 ou 2, dans lequel
R1 est un Y-phényle, Y-pyridyle, Y-thiényle, Y-furyle, Y-benzothiényle, Y-benzofuryle, où le phényle ou le radical hétéroaromatique peuvent être substitués une fois, deux fois ou trois fois par F, Cl, Br, CF₃, CN, OCF₃, O-(alkyle en C₁-C₆), un alkyle en C₁-C₆, (C₃-C₆)-cycloalkyle, N (R4) (R5), COOH, COO-(alkyle en C₁-C₆), CON(R6)(R7), CO(R12), et peuvent être substitués une fois par un (alkylène en C₀-C₁)-phényle, O-(C₀-C₁)-phényle, un pyrazolyle, un pyridyle, un thiényle, un furyle, un benzothiényle, un benzofuryle, où le radical hétéroaromatique ou le phényle peuvent eux-mêmes être substitués une fois, deux fois ou trois fois par F, Cl, Br, CF₃, CN, OCF₃, O-(alkyle en C₁-C₆), un alkyle en C₁-C₆, un cycloalkyle en C₃-C₆, N(R4a)(R5a), COOH, COO-(alkyle en C₁-C₆), CON (R6a) (R7a), CO(R12a) ;
R4, R5, R6, R7, R12, R4a, R5a, R6a, R7a, R12a sont indépendamment les uns des autres H, un
alkyle en C₁-C₈ ;
Y est -CH₂- ou -CH₂-CH₂-, qui peuvent être substitués une fois par CH₃ ;
R2 est un hydrogène, un alkyle en C₁-C₈, -CH₂-phényle, où le phényle peut facultativement être substitué, un cycloalkyle en C₃-C₈ ;
R3 est un alkyle en C₁-C₈, un phényle, un pyridyle, un thiényle, où le phényle, le pyridyle ou le thiényle peuvent facultativement être substitués, un cycloalkyle en C₃-C₈ ; ou
R2 et R3 conjointement avec les atomes de carbone portant ceux-ci forment un système cyclique monocyclique, saturé, à 6 à 7 chaînons dont les chaînons individuels peuvent être remplacés par un à trois atomes ou groupes atomiques de la série -CHR64-, -CR64R65- ;
R64, R65 sont, identiques ou différents, F, Cl, CF₃, OCF₃, un alkyle en C₁-C₆, O-(alkyle en C₁-C₆), N(R68)(R69), COOH, COO-(alkyle en C₁-C₆), CO-N(R70)(R71), CO (R76) ;
R68, R69, R70, R71, R76 sont, identiques ou différents, un hydrogène, un alkyle en C₁-C₆ ;
les formes tautomères des composés et leurs sels physiologiquement acceptables.

4. Composé de formule I selon les revendications 1 à 3, dans lequel
R2 est un isopropyle et
R3 est un méthyle.

5. Composé de formule I selon les revendications 1 à 3, dans lequel
R2 est un hydrogène et
R3 est un phényle, qui peut être substitué une fois par Cl.

6. Composé de formule I selon les revendications 1 à 3, dans lequel
R2 et R3 conjointement sont -CH₂-CH₂-CH₂-CH₂- ou -CH₂-CH₂-CH₂-CH₂-CH₂-.

7. Composé de formule I selon les revendications 1 à 3, dans lequel
R1 est un Y-phényle, Y-thiényle, Y-benzothiényle, où le phényle ou le radical hétéroaromatique peuvent être substitués une fois, deux fois ou trois fois par F, Cl, Br, CF₃, O-CH₃, -CH₃, -CH₂CH₃, -CH₂-CH₂-CH₂-CH₃, et être substitués une fois par un phényle, un pyrazolyle ou un thiényle,
où le radical hétéroaromatique ou le phényle peuvent eux-mêmes être substitués une fois, deux fois ou trois fois par F, Cl, Br, CF₃, O- (alkyle en C₁-C₆), un alkyle en C₁-C₆ ;
Y est -CH₂-, qui peut être substitué une fois par CH₃ ;
R2 est un hydrogène, un méthyle, un isopropyle, un cyclopropyle, un -CH₂-phényle, où le phényle peut être substitué par Cl à la position 4 ;
R3 est un méthyle, un phényle, un pyridyle, un cyclopropyle, où le phényle peut être substitué par Cl ; ou
R2 et R3 sont conjointement -CH₂-CH₂-CH₂-CH₂- ou -CH₂-CH₂-CH₂-CH₂-CH₂ ;
les formes tautomères des composés et leurs sels physiologiquement acceptables.

8. Médicament comprenant un ou plusieurs composés de formule I selon les revendications 1 à 7.

9. Utilisation des composés de formule I selon les revendications 1 à 7 pour la fabrication d'un médicament pour le traitement et/ou la prévention de troubles du métabolisme des acides gras et de troubles d'utilisation du glucose.

10. Utilisation des composés de formule I selon les revendications 1 à 7 pour la fabrication d'un médicament pour le traitement et/ou la prévention de troubles dans lesquels l'insulinorésistance est impliquée.

11. Utilisation des composés de formule I selon les revendications 1 à 7 pour la fabrication d'un médicament pour le traitement et/ou la prévention du diabète sucré et des séquelles associées à celui-ci.

12. Utilisation des composés de formule I selon les revendications 1 à 7 pour la fabrication d'un médicament pour le traitement et/ou la prévention de dyslipidémies et des séquelles de celles-ci.

13. Utilisation des composés de formule I selon les revendications 1 à 7 pour la fabrication d'un médicament pour le traitement et/ou la prévention de pathologies associées au syndrome métabolique.

14. Utilisation des composés de formule I selon les revendications 1 à 7 pour la fabrication d'un médicament pour le traitement et/ou la prévention de pathologies associées à un taux de HDL réduit.

15. Utilisation des composés de formule I selon les revendications 1 à 7 pour la fabrication d'un médicament pour le traitement et/ou la prévention de troubles athérosclérotiques.

16. Utilisation des composés de formule I selon les revendications 1 à 7 en combinaison avec au moins un principe actif supplémentaire pour la fabrication d'un médicament pour le traitement et/ou la prévention de troubles dans lesquels l'insulinorésistance est impliquée.

17. Procédé pour la fabrication d'un médicament comprenant un ou plusieurs des composés de formule I selon les revendications 1 à 7, qui comprend le mélange de ceux-ci avec un véhicule pharmaceutiquement adapté et la conversion de ce mélange en une forme adaptée pour administration.

18. Procédé de préparation de composés de formule I selon les revendications 1 à 7, dans lequel des dérivés de 3-oxo-isoxazole de formule II
a) sont acylés avec des chlorures de carbamoyle de formule III ;
ou
b) en deux étapes réagissent dans un premier temps avec du phosgène ou des équivalents tels que le chlorocarbonate de trichlorométhyle, le carbonate de ditrichlorométhyle ou le chloroformate de 4-nitrophényle et dans une seconde étape avec des amines de formule IV, ou
c) réagissent avec des isocyanates de formule V : O =C=N-R1.
où les substituants ont les définitions décrites ci-dessus.
